# EUROPÄISCHE PATENTSCHRIFT

(11) **EP 2 193 198 B1**
(45) Veröffentlichungstag und Bekanntmachung des Hinweises auf die Patenterteilung: **21.05.2014**
(21) Anmeldenummer: 08773420.8
(22) Anmeldetag: 12.06.2008
(51) Int. Cl.: C12N 15/55, C12N 9/82, A21D 8/04, A23L 1/015, A23L 1/217, A23L 1/305, A23F 5/02, A23F 5/16, A23F 5/22, A23G 1/02

(54) **HITZESTABILE ASPARAGINASE ZUR REDUKTION VON ACRYLAMID IN NAHRUNGS- ODER GENUSSMITTELN**
AMIDOHYDROLASES FOR PREPARING FOODSTUFFS OR STIMULANTS
AMINOHYDROLASES DESTINÉES À LA PRÉPARATION DE PRODUITS ALIMENTAIRES OU DE STIMULANTS

(30) Priorität: 13.06.2007 DE 102007027825
(43) Veröffentlichungstag der Anmeldung: 09.06.2010
(73) Patentinhaber: c-LEcta GmbH, 04103 Leipzig (DE)
(72) Erfinder: GREINER-STOEFFELE, Thomas, 04279 Leipzig (DE); STRUHALLA, Marc, 04229 Leipzig (DE)
(74) Vertreter: Bülle, Jan
(86) Internationale Anmeldenummer: PCT/EP2008/004742
(87) Internationale Veröffentlichungsnummer: WO 2008/151807

(56) Entgegenhaltungen:
- EP-A- 1 886 582
- WO-A-2006/128843
- WO-A-2008/110513
- WO-A1-2004/032648
- US-A- 5 719 056
- DATABASE EMBL [Online] 1. März 2002 (2002-03-01), "Pyrococcus furiosus DSM 3638, section 171 of 173 of the complete genome." XP002510347 gefunden im EBI accession no. EMBL:AE010296 Database accession no. AE010296
- LI J ET AL: "BIOSENSOR FOR ASPARAGINE USING A THERMOSTABLE RECOMBINANT ASPARAGINASE FROM ARCHAEOGLOBUS FULGIDUS", ANALYTICAL CHEMISTRY, AMERICAN CHEMICAL SOCIETY, US, vol. 74, no. 14, 15 July 2002 (2002-07-15) , pages 3336-3341, XP001132186, ISSN: 0003-2700, DOI: 10.1021/AC015653S
- PRITSA A A ET AL: "L-asparaginase of Thermus thermophilus: purification, properties and identification of essential amino acids for its catalytic activity.", MOLECULAR AND CELLULAR BIOCHEMISTRY JAN 2001, vol. 216, no. 1-2, January 2001 (2001-01), pages 93-101, ISSN: 0300-8177

### Sequenzprotokolle:

<110> c-Lecta GmbH
<120> Amidohydrolasen zur Aufbereitung von Nahrungs- oder Genussmitteln
<130> IV0003
<160> 6
<170> PatentIn version 3.4
<210> 1
   <211> 981
   <212> DNA
   <213> Pyrococcus furiosus
<400> 1
   gtgaaaattc ttctaattgg gatgggtgga acaattgcga gtgtaaaggg cgagaatgga      60
   tatgaggctt cgttgtccgt taaagaagtt ttagatatcg ccggaatcaa agattgtgag     120
   gattgtgatt ttctcgattt aaagaacgtt gatagcacgc ttatccagcc agaagattgg     180
   gtagatcttg ctgaaactct ttacaagaat gtaaaaaaat atgatggaat tatagtcact     240
   catggtaccg atactcttgc ctacacttct tcaatgataa gtttcatgct tagaaacccc     300
   ccaataccca tcgtatttac tggttctatg atacctgcca ctgaagaaaa tagtgatgcc     360
   cccctaaact tgcaaacagc aataaagttt gcaacttctg gaattagggg agtttacgtg     420
   gccttcaatg gaaaagttat gcttggagtt agaacatcta aggttaggac aatgagcaga     480
   gatgcattcg aaagcattaa ctaccctata attgcagaat taagaggaga agatctcgtg     540
   gttaacttta ttccaaagtt taacaatgga gaagtcacat tagaccttag gcacgatcca     600
   aaagttctag ttataaagct aatcccagga ctttcggggg acatatttag ggcagctgta     660
   gagctgggat atagaggaat tgtcatagaa ggttatggag ctggaggaat tccttatagg    720
   ggaagtgatt tacttcaaac aatagaggag ctctccaagg agattccaat agtaatgaca     780
   acccaggcaa tgtacgatgg agttgatcta acgaggtaca aagttgggag attagccctt     840
   agagctggag taatcccagc gggggacatg acaaaagagg caacagtaac aaagctcatg     900
   tggattctag gccacacaaa caatgtggaa gaaataaaag tattaatgag aaaaaatcta     960
   gttggagagc ttagagatta a                                                981
<210> 2
   <211> 326
   <212> PRT .
   <213> Pyrococcus furiosus
<400> 2
   Met Lys Ile Leu Leu Ile Gly Met Gly Gly Thr Ile Ala Ser Val Lys
   1 5 10 15
   Gly Glu Asn Gly Tyr Glu Ala Ser Leu Ser Val Lys Glu Val Leu Asp
   20 25 30
   Ile Ala Gly Ile Lys Asp Cys Glu Asp Cys Asp Phe Leu Asp Leu Lys
   35 40 45
   Asn Val Asp Ser Thr Leu Ile Gln Pro Glu Asp Trp Val Asp Leu Ala
   50 55 60
   Glu Thr Leu Tyr Lys Asn Val Lys Lys Tyr Asp Gly Ile Ile Val Thr
   65 70 75 80
   His Gly Thr Asp Thr Leu Ala Tyr Thr Ser Ser Met Ile Ser Phe Met
   85 90 95
   Leu Arg Asn Pro Pro Ile Pro Ile Val Phe Thr Gly Ser Met Ile Pro
   100 105 110
   Ala Thr Glu Glu Asn Ser Asp Ala Pro Leu Asn Leu Gln Thr Ala Ile
   115 120 125
   Lys Phe Ala Thr Ser Gly Ile Arg Gly Val Tyr Val Ala Phe Asn Gly
   130 135 140
   Lys Val Met Leu Gly Val Arg Thr Ser Lys Val Arg Thr Met Ser Arg
   145 150 155 160
   Asp Ala Phe Glu Ser Ile Asn Tyr Pro Ile Ile Ala Glu Leu Arg Gly
   165 170 175
   Glu Asp Leu Val Val Asn Phe Ile Pro Lys Phe Asn Asn Gly Glu Val
   180 185 190
   Thr Leu Asp Leu Arg His Asp Pro Lys Val Leu Val Ile Lys Leu Ile
   195 200 205
   Pro Gly Leu Ser Gly Asp Ile Phe Arg Ala Ala Val Glu Leu Gly Tyr
   210 215 220
   Arg Gly Ile Val Ile Glu Gly Tyr Gly Ala Gly Gly Ile Pro Tyr Arg
   225 230 235 240
   Gly Ser Asp Leu Leu Gln Thr Ile Glu Glu Leu Ser Lys Glu Ile Pro
   245 250 255
   Ile Val Met Thr Thr Gln Ala Met Tyr Asp Gly Val Asp Leu Thr Arg
   260 265 270
   Tyr Lys Val Gly Arg Leu Ala Leu Arg Ala Gly Val Ile Pro Ala Gly
   275 280 285
   Asp Met Thr Lys Glu Ala Thr Val Thr Lys Leu Met Trp Ile Leu Gly
   290 295 300
   His Thr Asn Asn Val Glu Glu Ile Lys Val Leu Met Arg Lys Asn Leu
   305 310 315 320
   Val Gly Glu Leu Arg Asp
   325
<210> 3
   <211> 40
   <212> DNA
   <213> Artificial
<220>
   <223> chemically synthesized
<400> 3
   acctgcggtc tcgcatgaaa attcttctaa ttgggatggg 40
<210> 4
   <211> 36
   <212> DNA
   <213> Artificial
<220>
   <223> chemically synthesized
<400> 4
   ggatccctgc agttaatctc taagctctcc aactag 36
<210> 5
   <211> 3669
   <212> DNA
   <213> Artificial
<220>
   <223> plasmid pRSF-1b
<400> 5
   tggtgtccgg gatctcgacg ctctccctta tgcgactcct gcattaggaa attaatacga 60
   ctcactatag gggaattgtg agcggataac aattcccctg tagaaataat tttgtttaac 120
   tttaataagg agatatacca tggcacatca ccaccaccat cacgtgggta ccggttcgaa 180
   tgatgacgac gacaagagtc cggatcccaa ttgggagctc gtgtacacgg cgcgcctgca 240
   ggtcgacaag cttgcggccg cactcgagtc tggtaaagaa accgctgctg cgaaatttga 300
   acgccagcac atggactcgt ctactagcgc agcttaatta acctaggctg ctgccaccgc 360
   tgagcaataa ctagcataac cccttggggc ctctaaacgg gtcttgaggg gttttttgct 420
   gaaacctcag gcatttgaga agcacacggt cacactgctt ccggtagtca ataaaccggt 480
   aaaccagcaa tagacataag cggctattta acgaccctgc cctgaaccga cgacaagctg 540
   acgaccgggt ctccgcaagt ggcacttttc ggggaaatgt gcgcggaacc cctatttgtt 600
   tatttttcta aatacattca aatatgtatc cgctcatgaa ttaattctta gaaaaactca 660
   tcgagcatca aatgaaactg caatttattc atatcaggat tatcaatacc atatttttga 720
   aaaagccgtt tctgtaatga aggagaaaac tcaccgaggc agttccatag gatggcaaga 780
   tcctggtatc ggtctgcgat tccgactcgt ccaacatcaa tacaacctat taatttcccc 840
   tcgtcaaaaa taaggttatc aagtgagaaa tcaccatgag tgacgactga atccggtgag 900
   aatggcaaaa gtttatgcat ttctttccag acttgttcaa caggccagcc attacgctcg 960
   tcatcaaaat cactcgcatc aaccaaaccg ttattcattc gtgattgcgc ctgagcgaga 1020
   cgaaatacgc ggtcgctgtt aaaaggacaa ttacaaacag gaatcgaatg caaccggcgc 1080
   aggaacactg ccagcgcatc aacaatattt tcacctgaat caggatattc ttctaatacc 1140
   tggaatgctg ttttcccggg gatcgcagtg gtgagtaacc atgcatcatc aggagtacgg 1200
   ataaaatgct tgatggtcgg aagaggcata aattccgtca gccagtttag tctgaccatc 1260
   tcatctgtaa catcattggc aacgctacct ttgccatgtt tcagaaacaa ctctggcgca 1320
   tcgggcttcc catacaatcg atagattgtc gcacctgatt gcccgacatt atcgcgagcc 1380
   catttatacc catataaatc agcatccatg ttggaattta atcgcggcct agagcaagac 1440
   gtttcccgtt gaatatggct catactcttc ctttttcaat attattgaag catttatcag 1500
   ggttattgtc tcatgagcgg atacatattt gaatgtattt agaaaaataa acaaataggc 1560
   atgcagcgct cttccgcttc ctcgctcact gactcgctac gctcggtcgt tcgactgcgg 1620
   cgagcggtgt cagctcactc aaaagcggta atacggttat ccacagaatc aggggataaa 1680
   gccggaaaga acatgtgagc aaaaagcaaa gcaccggaag aagccaacgc cgcaggcgtt 1740
   tttccatagg ctccgccccc ctgacgagca tcacaaaaat cgacgctcaa gccagaggtg 1800
   gcgaaacccg acaggactat aaagatacca ggcgtttccc cctggaagct ccctcgtgcg 1860
   ctctcctgtt ccgaccctgc cgcttaccgg atacctgtcc gcctttctcc cttcgggaag 1920
   cgtggcgctt tctcatagct cacgctgttg gtatctcagt tcggtgtagg tcgttcgctc 1980
   caagctgggc tgtgtgcacg aaccccccgt tcagcccgac cgctgcgcct tatccggtaa 2040
   ctatcgtctt gagtccaacc cggtaagaca cgacttatcg ccactggcag cagccattgg 2100
   taactgattt agaggacttt gtcttgaagt tatgcacctg ttaaggctaa actgaaagaa 2160
   cagattttgg tgagtgcggt cctccaaccc acttaccttg gttcaaagag ttggtagctc 2220
   agcgaacctt gagaaaacca ccgttggtag cggtggtttt tctttattta tgagatgatg 2280
   aatcaatcgg tctatcaagt caacgaacag ctattccgtt actctagatt tcagtgcaat 2340
   ttatctcttc aaatgtagca cctgaagtca gccccatacg atataagttg taattctcat 2400
   gttagtcatg ccccgcgccc accggaagga gctgactggg ttgaaggctc tcaagggcat 2460
   cggtcgagat cccggtgcct aatgagtgag ctaacttaca ttaattgcgt tgcgctcact 2520
   gcccgctttc cagtcgggaa acctgtcgtg ccagctgcat taatgaatcg gccaacgcgc 2580
   ggggagaggc ggtttgcgta ttgggcgcca gggtggtttt tcttttcacc agtgagacgg 2640
   gcaacagctg attgcccttc accgcctggc cctgagagag ttgcagcaag cggtccacgc 2700
   tggtttgccc cagcaggcga aaatcctgtt tgatggtggt taacggcggg atataacatg 2760
   agctgtcttc ggtatcgtcg tatcccacta ccgagatgtc cgcaccaacg cgcagcccgg 2820
   actcggtaat ggcgcgcatt gcgcccagcg ccatctgatc gttggcaacc agcatcgcag 2880
   tgggaacgat gccctcattc agcatttgca tggtttgttg aaaaccggac atggcactcc 2940
   agtcgccttc ccgttccgct atcggctgaa tttgattgcg agtgagatat ttatgccagc 3000
   cagccagacg cagacgcgcc gagacagaac ttaatgggcc cgctaacagc gcgatttgct 3060
   ggtgacccaa tgcgaccaga tgctccacgc ccagtcgcgt accgtcttca tgggagaaaa 3120
   taatactgtt gatgggtgtc tggtcagaga catcaagaaa taacgccgga acattagtgc 3180
   aggcagcttc cacagcaatg gcatcctggt catccagcgg atagttaatg atcagcccac 3240
   tgacgcgttg cgcgagaaga ttgtgcaccg ccgctttaca ggcttcgacg ccgcttcgtt 3300
   ctaccatcga caccaccacg ctggcaccca gttgatcggc gcgagattta atcgccgcga 3360
   caatttgcga cggcgcgtgc agggccagac tggaggtggc aacgccaatc agcaacgact 3420
   gtttgcccgc cagttgttgt gccacgcggt tgggaatgta attcagctcc gccatcgccg 3480
   cttccacttt ttcccgcgtt ttcgcagaaa cgtggctggc ctggttcacc acgcgggaaa 3540
   cggtctgata agagacaccg gcatactctg cgacatcgta taacgttact ggtttcacat 3600
   tcaccaccct gaattgactc tcttccgggc gctatcatgc cataccgcga aaggttttgc 3660
   gccattcga 3669
<210> 6
   <211> 4554
   <212> DNA
   <213> Artificial
<220>
   <223> Plasmid pRSF_PF-AsnI
<400> 6
   tggtgtccgg gatctcgacg ctctccctta tgcgactcct gcattaggaa attaatacga 60
   ctcactatag gggaattgtg agcggataac aattcccctg tagaaataat tttgtttaac 120
   tttaataagg agatatacca tgaaaattct tctaattggg atgggtggaa caattgcgag 180
   tgtaaagggc gagaatggat atgaggcttc gttgtccgtt aaagaagttt tagatatcgc 240
   cggaatcaaa gattgtgagg attgtgattt tctcgattta aagaacgttg atagcacgct 300
   tatccagcca gaagattggg tagatcttgc tgaaactctt tacaagaatg taaaaaaata 360
   tgatggaatt atagtcactc atggtaccga tactcttgcc tacacttctt caatgataag 420
   tttcatgctt agaaaccccc caatacccat cgtatttact ggttctatga tacctgccac 480
   tgaagaaaat agtgatgccc ccctaaactt gcaaacagca ataaagtttg caacttctgg 540
   aattagggga gtttacgtgg ccttcaatgg aaaagttatg cttggagtta gaacatctaa 600
   ggttaggaca atgagcagag atgcattcga aagcattaac taccctataa ttgcagaatt 660
   aagaggagaa gatctcgtgg ttaactttat tccaaagttt aacaatggag aagtcacatt 720
   agaccttagg cacgatccaa aagttctagt tataaagcta atcccaggac tttcggggga 780
   catatttagg gcagctgtag agctgggata tagaggaatt gtcatagaag gttatggagc 840
   tggaggaatt ccttataggg gaagtgattt acttcaaaca atagaggagc tctccaagga 900
   gattccaata gtaatgacaa cccaggcaat gtacgatgga gttgatctaa cgaggtacaa 960
   agttgggaga ttagccctta gagctggagt aatcccagcg ggggacatga caaaagaggc 1020
   aacagtaaca aagctcatgt ggattctagg ccacacaaac aatgtggaag aaataaaagt 1080
   attaatgaga aaaaatctag ttggagagct tagagattaa ctgcaggtcg acaagcttgc 1140
   ggccgcactc gagtctggta aagaaaccgc tgctgcgaaa tttgaacgcc agcacatgga 1200
   ctcgtctact agcgcagctt aattaaccta ggctgctgcc accgctgagc aataactagc 1260
   ataacccctt ggggcctcta aacgggtctt gaggggtttt ttgctgaaac ctcaggcatt 1320
   tgagaagcac acggtcacac tgcttccggt agtcaataaa ccggtaaacc agcaatagac 1380
   ataagcggct atttaacgac cctgccctga accgacgaca agctgacgac cgggtctccg 1440
   caagtggcac ttttcgggga aatgtgcgcg gaacccctat ttgtttattt ttctaaatac 1500
   attcaaatat gtatccgctc atgaattaat tcttagaaaa actcatcgag catcaaatga 1560
   aactgcaatt tattcatatc aggattatca ataccatatt tttgaaaaag ccgtttctgt 1620
   aatgaaggag aaaactcacc gaggcagttc cataggatgg caagatcctg gtatcggtct 1680
   gcgattccga ctcgtccaac atcaatacaa cctattaatt tcccctcgtc aaaaataagg 1740
   ttatcaagtg agaaatcacc atgagtgacg actgaatccg gtgagaatgg caaaagttta 1800
   tgcatttctt tccagacttg ttcaacaggc cagccattac gctcgtcatc aaaatcactc 1860
   gcatcaacca aaccgttatt cattcgtgat tgcgcctgag cgagacgaaa tacgcggtcg 1920
   ctgttaaaag gacaattaca aacaggaatc gaatgcaacc ggcgcaggaa cactgccagc 1980
   gcatcaacaa tattttcacc tgaatcagga tattcttcta atacctggaa tgctgttttc 2040
   ccggggatcg cagtggtgag taaccatgca tcatcaggag tacggataaa atgcttgatg 2100
   gtcggaagag gcataaattc cgtcagccag tttagtctga ccatctcatc tgtaacatca 2160
   ttggcaacgc tacctttgcc atgtttcaga aacaactctg gcgcatcggg cttcccatac 2220
   aatcgataga ttgtcgcacc tgattgcccg acattatcgc gagcccattt atacccatat 2280
   aaatcagcat ccatgttgga atttaatcgc ggcctagagc aagacgtttc ccgttgaata 2340
   tggctcatac tcttcctttt tcaatattat tgaagcattt atcagggtta ttgtctcatg 2400
   agcggataca tatttgaatg tatttagaaa aataaacaaa taggcatgca gcgctcttcc 2460
   gcttcctcgc tcactgactc gctacgctcg gtcgttcgac tgcggcgagc ggtgtcagct 2520
   cactcaaaag cggtaatacg gttatccaca gaatcagggg ataaagccgg aaagaacatg 2580
   tgagcaaaaa gcaaagcacc ggaagaagcc aacgccgcag gcgtttttcc ataggctccg 2640
   cccccctgac gagcatcaca aaaatcgacg ctcaagccag aggtggcgaa acccgacagg 2700
   actataaaga taccaggcgt ttccccctgg aagctccctc gtgcgctctc ctgttccgac 2760
   cctgccgctt accggatacc tgtccgcctt tctcccttcg ggaagcgtgg cgctttctca 2820
   tagctcacgc tgttggtatc tcagttcggt gtaggtcgtt cgctccaagc tgggctgtgt 2880
   gcacgaaccc cccgttcagc ccgaccgctg cgccttatcc ggtaactatc gtcttgagtc 2940
   caacccggta agacacgact tatcgccact ggcagcagcc attggtaact gatttagagg 3000
   actttgtctt gaagttatgc acctgttaag gctaaactga aagaacagat tttggtgagt 3060
   gcggtcctcc aacccactta ccttggttca aagagttggt agctcagcga accttgagaa 3120
   aaccaccgtt ggtagcggtg gtttttcttt atttatgaga tgatgaatca atcggtctat 3180
   caagtcaacg aacagctatt ccgttactct agatttcagt gcaatttatc tcttcaaatg 3240
   tagcacctga agtcagcccc atacgatata agttgtaatt ctcatgttag tcatgccccg 3300
   cgcccaccgg aaggagctga ctgggttgaa ggctctcaag ggcatcggtc gagatcccgg 3360
   tgcctaatga gtgagctaac ttacattaat tgcgttgcgc tcactgcccg ctttccagtc 3420
   gggaaacctg tcgtgccagc tgcattaatg aatcggccaa cgcgcgggga gaggcggttt 3480
   gcgtattggg cgccagggtg gtttttcttt tcaccagtga gacgggcaac agctgattgc 3540
   ccttcaccgc ctggccctga gagagttgca gcaagcggtc cacgctggtt tgccccagca 3600
   ggcgaaaatc ctgtttgatg gtggttaacg gcgggatata acatgagctg tcttcggtat 3660
   cgtcgtatcc cactaccgag atgtccgcac caacgcgcag cccggactcg gtaatggcgc 3720
   gcattgcgcc cagcgccatc tgatcgttgg caaccagcat cgcagtggga acgatgccct 3780
   cattcagcat ttgcatggtt tgttgaaaac cggacatggc actccagtcg ccttcccgtt 3840
   ccgctatcgg ctgaatttga ttgcgagtga gatatttatg ccagccagcc agacgcagac 3900
   gcgccgagac agaacttaat gggcccgcta acagcgcgat ttgctggtga cccaatgcga 3960
   ccagatgctc cacgcccagt cgcgtaccgt cttcatggga gaaaataata ctgttgatgg 4020
   gtgtctggtc agagacatca agaaataacg ccggaacatt agtgcaggca gcttccacag 4080
   caatggcatc ctggtcatcc agcggatagt taatgatcag cccactgacg cgttgcgcga 4140
   gaagattgtg caccgccgct ttacaggctt cgacgccgct tcgttctacc atcgacacca 4200
   ccacgctggc acccagttga tcggcgcgag atttaatcgc cgcgacaatt tgcgacggcg 4260
   cgtgcagggc cagactggag gtggcaacgc caatcagcaa cgactgtttg cccgccagtt 4320
   gttgtgccac gcggttggga atgtaattca gctccgccat cgccgcttcc actttttccc 4380
   gcgttttcgc agaaacgtgg ctggcctggt tcaccacgcg ggaaacggtc tgataagaga 4440
   caccggcata ctctgcgaca tcgtataacg ttactggttt cacattcacc accctgaatt 4500
   gactctcttc cgggcgctat catgccatac cgcgaaaggt tttgcgccat tcga 4554

## Beschreibung

Die Erfindung betrifft die Verwendung einer Amidohydrolase zur Aufbereitung eines Nahrungsmittels oder eines Genussmittels.

Kohlenhydrathaltige Nahrungsmittel, werden seit Jahrtausenden von Menschen auf der ganzen Welt verzehrt. Derartige Nahrungsmittel sind in der heutigen Zeit in vielfältigster Weise erhältlich, beispielsweise in Form von Knäckebrot, Zwieback, Keksen, Brezeln, Toastbrot, Frühstückscerealien, Biskuites, Kartoffelchips, Tortillachips, Pommes frites, Reiswaffeln, etc.

Bei der Verarbeitung, Zubereitung oder Aufbereitung von kohlenhydrathaltigen Nahrungsmitteln, insbesondere bei Erhitzungsprozessen, wie das beispielsweise beim Backen, Braten, Rösten, Grillen oder Frittieren der Fall ist, wird häufig Acrylamid gebildet, welches sich in der Folge in diesen Nahrungsmitteln anreichert. Ein ähnliches Phänomen wird auch bei der Aufbereitung von Genussmitteln, wie beispielsweise bei der Zubereitung von Kaffee beobachtet.

Im April 2002 wurden von der schwedischen Behörde für Lebensmittelsicherheit erstmals Untersuchungsergebnisse zur Acrylamidbelastung von Nahrungsmitteln veröffentlicht. Im selben Jahr veröffentlichte die Weltgesundheitsorganisation (WHO) einen Bericht, der unter anderem gesundheitliche Risiken diskutiert, die mit einer hohen Acrylamidbelastung in Nahrungsmitteln einhergehen können (FAO/WHO: *"*Health Implications of Acrylamide in Food", Geneva, 2002).

Acrylamid ist eine Substanz, die direkt das menschliche Erbgut (DNA) angreift. Ferner wird Acrylamid von Leberenzymen in Glycidamid umgesetzt, welchem eine genotoxische Wirkung zugeschrieben wird. Acrylamid wie auch Glycidamid bilden Verbindungen mit Aminosäuren und Nukleinbasen und können so die Struktur und Funktion von beispielsweise der DNA und Hämoglobin verändern. Insgesamt wird Acrylamid von Experten als krebserzeugend, erbgutverändernd, giftig, reizend, sensibilislerend und fortpflanzungsgefährdend eingestuft.

Der wichtigste Ausgangsstoff zur Entstehung von Acrylamid in Nahrungsmitteln ist die Aminosäure Asparagin, die ubiquitär in Nahrungsmitteln wie beispielsweise Kartoffeln, Reis und Getreide, aber auch in Kaffee, Trockenfrüchten in mehr oder minder hohen Konzentrationen vorkommt. Enthält das Nahrungs-/Genussmittel neben Asparagin auch noch Zucker wie z. B. Fructose oder Glucose, so wird die Bildung von Acrylamid bei hohen Temperaturen noch zusätzlich gefördert.

Aufbereitungsverfahren für Nahrungs- oder Genussmittel, welche Vorbehandlungsschritte zur Reduktion des Acrylamidgehalts beinhalten, sind bereits aus dem Stand der Technik bekannt (vgl. z.B. US 7,037,540, US 2004/81724 oder US 2005/ 202153). Aus dem Stand der Technik sind auch Verfahren bekannt, bei denen zur Vorbehandlung Enzyme, insbesondere Asparaginasen eingesetzt werden (vgl. z.B. WO 2006/128843, WO 2008/110513 oder WO 2004/032648). Diese Vorbehandlungsschritte, welche die Entfernung, Inaktivierung und/oder die Extraktion von Asparagin aus den aufzubereitenden Nahrungs- oder Genussmitteln erleichtern sollen, sind teilweise sehr kosten- und/oder zeitaufwändig.

So umfassen beispielsweise Verfahren zur Aufbereitung von Kaffeebohnen jeweils aufwändige Trocknungs-, Bedampfungs- oder Befeuchtungsschritte. Durch derartige Vorbehandlungsschritte sollen die Poren der Kaffeebohnen geöffnet werden, um in der Folge das in den Kaffeebohnen enthaltene Asparagin besser extrahieren, reduzieren oder inaktivieren zu können.

Bei einer Trocknung werden die Kaffeebohnen bei Temperaturen unter ca. 50°C erwärmt, um anschließend in Asparagin-inaktivierenden Lösungen, wie beispielsweise Calciumlactat oder Calciumcitrat eingeweicht zu werden.

Bei einem Bedampfen/Befeuchten werden die Kaffeebohnen mit Niederdruck- oder atmosphärischem Dampf oder mit Wasser besprüht, wobei die Feuchtigkeit von den Bohnen aufgenommen wird. Daran anschließend werden die Bohnen - in einem separaten Schritt - üblicherweise mit Asparagin-inaktivierenden Lösungen behandelt.

All diese vorstehend aufgeführten, aufwändigen und zum Teil sehr zeitintensiven Vorbehandlungsschritte führen direkt oder indirekt zu erhöhten Kosten, da sie den gesamten Aufbereitungsprozess verlängern und in der Folge auch verteuern.

Der Erfindung liegt somit die Aufgabe zugrunde, die aus dem Stand der Technik bekannten Aufbereitungsverfahren von Nahrungsmitteln oder Genussmitteln zu verbessern. Durch derartige Verbesserungen sollten insbesondere Vorbehandlungsschritte zur Reduktion des Gehalts an Asparagin bzw. Acrylamid in Nahrungs- oder Genussmitteln vereinfacht werden, um so das gesamte Aufbereitungsverfahren kürzer und kostengünstiger zu gestalten.

Diese Aufgabe wird durch den Gegenstand der Patentansprüche gelöst.

Es wurde überraschend gefunden, dass Nahrungs- oder Genussmittel unter Verwendung einer Amidohydrolase, welche nach einer Inkubationsdauer von 20 min bei 70°C eine Restaktivität von wenigstens 75% aufweist, aufbereitet werden können.

Die Erfindung betrifft die Verwendung einer Amidohydrolase. vorzugsweise Asparaginase, welche nach einer Inkubationsdauer von 20 min bei 70°C eine Restaktivität von wenigstens 75% aufweist und ein Temperatur-Optimum im Bereich von 70 bis 120°C aufweist, zur Aufbereitung eines Nahrungsmittels oder eines Genussmittels, vorzugsweise zur Verringerung des Gehalts an Asparagin in dem Nahrungs- oder Genussmittel durch Inkubieren des Nahrungsmittels oder Genussmittels mit der Amidohydrolase bei einer Inkubationstemperatur von wenigstens 70°C, wobei die Amidohydrolase eine Asparaginase ist, deren Aminosäuresequenz eine Homologie von wenigstens 50% zur Aminosäuresequenz <SEQ ID NO: 2> aufweist. Durch die Verringerung des Gehalts an Asparagin wird vorzugsweise auch der Gehalt an Acrylamid in dem Nahrungs- oder Genussmittel verringert, da Asparagin ein *Precursor* von Acrylamid ist, wenn das Nahrungs- oder Genussmittel einer nachfolgenden thermischen Behandlung unterzogen wird.
Figur 1 zeigt die Eichgerade der Ammoniumionenbestimmung mittels Nessler-Reagens. x-Achse: Menge NH₄ pro 40µl, y-Achse: Optische Dichte (OD) bei 436 nm. Der Korrelationskoeffizient R² beträgt 0,9979.
Figur 2 zeigt die enzymatische Aktivität der Asparaginase I von *Pyrococcus furiosus* bei unterschiedlichen Inkubationstemperaturen. rA: relative Aktivität (in %), T [°C]: Temperatur in Grad Celsius.
Figur 3 zeigt die Temperaturstabilität der Asparaginase I von *Pyrococcus furiosus* bei einer Temperatur von 95°C. rA: relative Aktivität (in %), min: Zeit in Minuten. Aus der Grafik ist ersichtlich, dass die Asparaginase bei 95°C nach einer Inkubationsdauer von 60 Minuten eine relative Aktivität bzw. Restaktivität von ca. 100% aufweist.
Figur 4 zeigt die Temperaturstabilität der Asparaginase I von *Pyrococcus furiosus* bei einer Temperatur von 99°C. rA: relative Aktivität (in %), min: Zeit in Minuten. Aus der Grafik ist ersichtlich, dass die Asparaginase bei 99°C nach einer Inkubationsdauer von 60 Minuten eine relative Aktivität bzw. Restaktivität von ca. 70% aufweist.

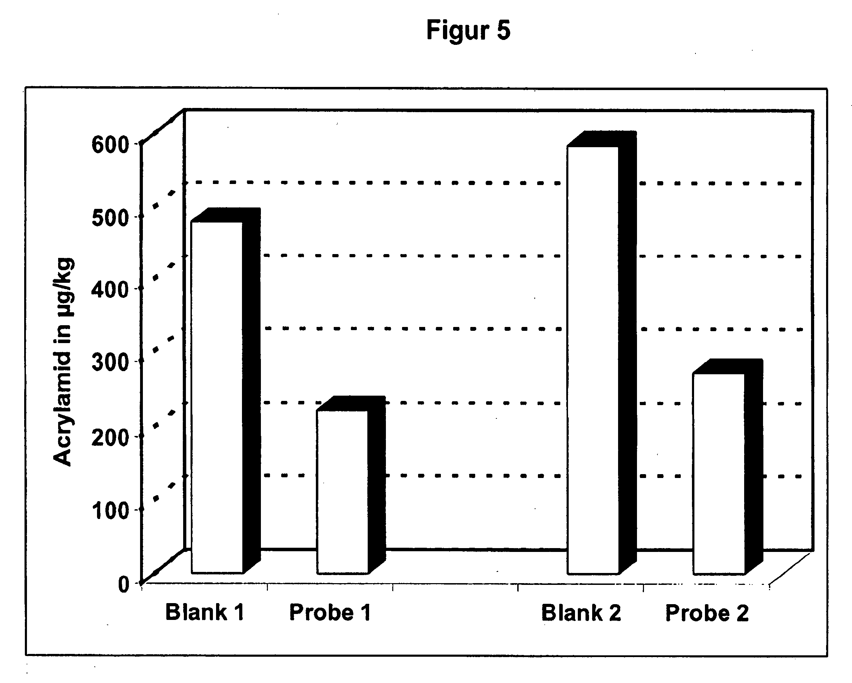

Figur 5 zeigt die Reduktion des Acrylamidgehaltes nach der Röstung von zwei Kaffeesorten durch Vorbehandlung der Rohkaffeebohnen mit der erfindungsgemäßen Asparaginase gemäß Beispiel 4 bei 80 °C. Blank 1 und Probe 1 - Kaffeesorte Arabica-Mischung, Blank 2 und Probe 2 - Kaffeesorte Brazil Arabica.

Amidohydrolasen gehören zur Enzymfamilie der Hydrolasen. Amidohydrolasen zeichnen sich dadurch aus, dass sie Amid-Gruppen spalten/hydrolisieren. Sie sind unter den EC-Nummem EC 3.5.1 und 3.5.2. zusammengefasst (*Enzyme Commission number,* nach der Definition des *Nomenclature Committee of the International Union of Biochemistry and Molecular Biology* (NC-IUBMB)).

Die DNA- und Polypeptidsequenz der Asparaginase I von *Pyrococcus furiosus* sind bereits bekannt, z.B. aus der Datenbank des Europäischen Instituts für Bioinformatik des Europäischen Laboratoriums für Molekularbiologie (vgl. EMBL-EBI, *Pyrococcus furiosus* DSM 3638, Zugriffsnummer AE010296).

Unter dem Begriff "Restaktivität" im Sinne der Beschreibung ist jene spezifische/ volumetrische enzymatische Aktivität zu verstehen, welche ein Enzym nach einer bestimmten Inkubationsdauer bei einer bestimmten Temperatur, verglichen mit der ursprünglichen spezifischen/volumetrischen Aktivität im Bereich seines Temperatur-Optimums, unter ansonsten identischen Reaktionsbedingungen (pH, Substrat, etc.) aufweist. Dabei ist unter der spezifischen/volumetrischen Aktivität eines Enzyms im Sinne der Beschreibung eine bestimmte Menge eines umgesetzten Substrats (in µmol) pro Zeit (in min) pro Enzymmenge (in mg bzw. ml) zu verstehen. Die Restaktivität eines Enzyms ergibt sich aus der spezifischen/volumetrischen Aktivität des Enzyms nach der o.g. Inkubationsdauer dividiert durch die ursprüngliche spezifische/volumetrische Aktivität, ausgedrückt in Prozent (%). Die spezifische Aktivität eines Enzyms wird hierbei vorzugsweise in U/mg, die volumetrische Aktivität eines Enzyms vorzugsweise in U/ml angegeben. Alternativ kann die spezifische/ volumetrische Aktivität eines Enzyms im Sinne der Beschreibung auch in katal/mg bzw. katal/ml angegeben sein.

Der Begriff "enzymatische Wirksamkeit", der manchmal auch als "katalytische Wirksamkeit" oder "katalytische Effizienz" bezeichnet wird, ist dem Fachmann allgemein bekannt und bezieht sich auf die Umsatzgeschwindigkeit eines Enzyms und wird üblicherweise über den Quotienten von Kₖₐₜ/K_{M} ausgedrückt werden, wobei kₖₐₜ die katalytische Konstante (auch *turnover number* bezeichnet) und der K_{M}-Wert jener Substratkonzentration entspricht, bei dem die Reaktionsgeschwindigkeit bei der Hälfte ihres Maximalwertes liegt. Die enzymatische Wirksamkeit eines Enzyms kann alternativ auch durch die spezifische Aktivität (µmol umgesetztes Substrat x mg⁻¹ x min⁻¹; vgl. oben) oder die volumetrische Aktivität (µmol umgesetztes Substrat x ml⁻¹ x min⁻¹; vgl. oben) angegeben werden. Im Zusammenhang mit der enzymatischen Wirksamkeit kann auf die allgemeine Literatur wie beispielsweise Voet et al. "Biochemie", 1992, VCH-Verlag, Kapitel 13, Seiten 331-332 verwiesen werden.

In bevorzugten Ausführungsformen A₁-A₇ bis F₁-F₇ weist die erfindungsgemäß verwendete Amidohydrolase unter den in der nachstehenden Tabelle angegebenen Bedingungen eine Restaktivität von vorzugsweise wenigstens 75%, bevorzugter wenigstens 80%, am bevorzugtesten wenigstens 90% auf:

| Nr. | Dauer | Temperatur (°C) | | | | | |
|---|---|---|---|---|---|---|---|
| | | A | B | C | D | E | F |
| 1 | 5 min | 50 | 60 | 70 | 80 | 90 | 100 |
| 2 | 10 min | 50 | 60 | 70 | 80 | 90 | 100 |
| 3 | 20 min | 50 | 60 | 70 | 80 | 90 | 100 |
| 4 | 30 min | 50 | 60 | 70 | 80 | 90 | 100 |
| 5 | 40 min | 50 | 60 | 70 | 80 | 90 | 100 |
| 6 | 50 min | 50 | 60 | 70 | 80 | 90 | 100 |
| 7 | 60 min | 50 | 60 | 70 | 80 | 90 | 100 |

In vorstehender Tabelle bedeutet beispielsweise Ausführungsform C₆, dass die Amidohydrolase nach mindestens 50 Minuten bei 70°C eine Restaktivität von wenigstens 75%, bevorzugter wenigstens 80%, am bevorzugtesten wenigstens 90% aufweist. In einer besonders bevorzugten Ausführungsform weist die Amidohydrolase unter den vorstehend aufgeführten Bedingungen eine Restaktivität im Bereich von vorzugsweise 75 - 100%, bevorzugter 75 - 90% auf.

Bei der eingesetzten Amidohydrolase handelt es sich um eine Asparaginase. Unter einer Asparaginase im Sinne der Beschreibung wird ein Enzym verstanden, welches die Hydrolyse von Asparagin zu Aspartat und Ammonium katalysiert. In einer bevorzugten Ausführungsform handelt es sich um eine Asparaginase vom Typ I, in einer anderen bevorzugten Ausführungsform handelt es sich um eine Asparaginase vom Typ II.

Vorzugsweise ist die Amidohydrolase, d.h. die Asparaginase, thermoaktiv.

"Thermoaktiv" im Sinne der Beschreibung sind solche Amidohydrolasen, d.h. Asparaginasen, deren Temperatur-Optimum oberhalb von 50°C liegt.

Der Begriff "Temperatur-Optimum" ist dem Fachmann allgemein bekannt und betrifft jenen Temperaturbereich, bei dem ein Enzym seine maximale enzymatische Wirksamkeit aufweist. In diesem Zusammenhang kann auf die einschlägige Literatur, wie beispielsweise Voet et al. "Biochemie", 1992, VCH-Verlag, Kapitel 13, Seite 331; I.H. Segel, Enzyme Kinetics: Behavior and Analysis of Rapid Equilibrium and Steady- ' State Enzyme Systems, Wiley Interscience, 1993; und A.G. Marangoni, Enzyme Kinetics: A Modem Approach, Wiley Interscience, 2002.

Vorzugsweise ist im Sinne der Beschreibung unter dem Temperatur-Optimum jener Temperaturbereich zu verstehen, in dem die erfindungsgemäß verwendete Amidohydrolase wenigstens 80%, vorzugsweise wenigstens 90% der maximalen enzymatischen Wirksamkeit, bei ansonsten gleichbleibenden Reaktionsbedingungen. aufweist.

Das Temperatur-Optimum der Amidohydrolase, d.h. der Asparaginase, liegt im Bereich von 70 bis 120°C, bevorzugter im Bereich von 75 bis 110°C, nach bevorzugter im Bereich von 80 bis 100°C und am bevorzugtesten im Bereich von 85 bis 95°C.

Bevorzugt sind die erfindungsgemäß eingesetzten Amidohydrolasen, d.h. die Asparaginasen, daher nicht nur thermostabil (d.h. halten hinsichtlich ihrer enzymatischen Aktivität einer thermischen Behandlung stand), sondern sind zusätzlich thermoaktiv (d.h. entfalten ihre volle enzymatische Aktivität erst bei erhöhter Temperatur).

Aus dem Stand der Technik sind bisher keine Verfahren bekannt, in denen eine thermoaktive Asparaginase zur Behandlung von Nahrungs- oder Genussmitteln zur Reduktion des Acrylamid-Gehalts eingesetzt wurden.

In einer bevorzugten Ausführungsform weist die Amidohydrolase, d.h. Asparaginase, bei einer Temperatur von vorzugsweise 60 bis 120°C, bevorzugter 65 bis 110°C, noch bevorzugter 70 bis 100°C, am bevorzugtesten 75 bis 100°C und insbesondere 80 bis 90°C eine spezifische Aktivität von vorzugsweise wenigstens 100, bevorzugter wenigstens 200, noch bevorzugter wenigstens 300, noch bevorzugter wenigstens 500, am bevorzugtesten wenigstens 800 und insbesondere wenigstens 1000 Units/mg auf, wobei 1 Unit als diejenige Menge an Amidohydrolase definiert ist, welche 1,0 µmol Ammoniak pro Minute aus L-Asparagin bei der entsprechenden Reaktionstemperatur und einem pH-Wert von 8.6 (50 mM Tris-HCl, pH-Einstellung bei 25 °C) freisetzt.

Es wurde überraschend gefunden, dass thermoaktive Asparaginasen gegenüber anderen Asparaginasen erhebliche Vorteile aufweisen. So kann mit Hilfe von thermoaktiven Asparaginasen die Spaltung des Asparagins bei vergleichsweise hohen Temperaturen durchgeführt werden, wodurch eine Kompatibilität erreicht wird mit Prozessen, bei denen hohe Temperaturen, insbesondere Halteprozesse bei hohen Temperaturen, ohnehin eine Rolle spielen. Ferner erlaubt die Durchführung der Spaltung des Asparagins bei höheren Temperaturen eine höhere Reaktionsgeschwindigkeit.

Bei der enzymatischen Behandlung von Kaffeebohnen ist es beispielsweise erforderlich, die grünen Kaffeebohnen mit Wasser quellen zu lassen, ehe sie mit Enzym behandelt werden können. Um die Kaffebohnen dann im Anschluss rösten zu können, müssen sie zuvor wieder getrocknet werden. Die enzymatische Behandlung von Kaffee erfordert daher u.a. die folgenden Schritte: a) Anfeuchten; b) enzymatische Behandlung im angefeuchteten Zustand; c) Trocknen; d) Rösten.

Übliche industrielle Verfahren zur Entkoffeinierung bzw. zur Gewährleistung des "milden Aromas" umfassen bereits ohnehin die oben genannten Verfahrensschritte a), c) und d).

Für den Trocknungsschritt c) müssen die Kaffeebohnen auf eine ausreichende Temperatur erwärmt werden, da das Wasser sonst nicht effizient entfernt werden kann. Die Erwärmung und Befeuchtung der Kaffeebohnen erfolgt bevorzugt mit heißem Wasserdampf (>100°C). Der anschließende Trocknungsschritt c) läuft dann bei 70-80°C ab.

Hätte die eingesetzte Amidohydrolase ein Temperatur-Optimum von z.B. lediglich 50°C, so wäre dies sehr ungünstig, da man zunächst zur Enzymbehandlung auf 50°C abkühlen und anschließend wieder zum Trocknen aufheizen müsste.

Mit Hilfe der erfindungsgemäß bevorzugten, thermoaktiven Asparaginasen ist hingegen kein Abkühlen erforderlich, was einen besonderen Vorteil der Erfindung darstellt.

Ein weiterer Vorteil beim Einsatz thermoaktiver Asparaginasen ist darin zu sehen, dass infolge der erhöhten Temperatur auch die Diffusion des zu hydrolysierenden Asparagins erhöht ist, was sich ebenfalls in einer verbesserten Effizienz des erfindungsgemäßen Verfahrens niederschlägt.

Das erfindungsgemäße Verfahren ermöglicht es, unter Beibehaltung der Prozessschritte üblicher Verfahren zur Aufbereitung eines Nahrungsmittels oder eines Genussmittels eine Aufbereitung mit Hilfe einer Amidohydrolase zu erreichen, d.h. ohne gravierende Veränderungen der Verfahrensabläufe. Bevorzugt kann die enzymatische Behandlung während eines Prozessschritts durchgeführt werden, bei dem das Nahrungsmittel bzw. das Genussmittel ohnehin einer erhöhten Temperatur ausgesetzt ist.

In einer weiteren bevorzugten Ausführungsform weist die Amidohydrolase, d.h. die Asparaginase, bei einer Temperatur von vorzugsweise 60 bis 120°C, bevorzugter 65 bis 110°C, noch bevorzugter 70 bis 100°C, am bevorzugtesten 75 bis 100°C und insbesondere 80-90°C eine volumetrische Aktivität von vorzugsweise wenigstens 50, bevorzugter wenigstens 100, noch bevorzugter wenigstens 300, noch bevorzugter wenigstens 500, am bevorzugtesten wenigstens 800 und insbesondere wenigstens 1000 Units/ml auf, wobei 1 Unit als diejenige Menge an Amidohydrolase definiert ist, welche 1,0 µmol Ammoniak pro Minute aus L-Asparagin bei der entsprechenden Reaktionstemperatur und einem pH-Wert von 8,6 (50 mM Tris-HCl, pH-Einstellung bei 25 °C) freisetzt.

In einer bevorzugten Ausführungsform weist die Amidohydrolase, d.h. die Asparaginase, ein pH-Optimum im Bereich von vorzugsweise pH 1 bis pH 14, bevorzugter im Bereich von pH 3 bis pH 12, noch bevorzugter im Bereich von pH 5 bis pH 11, am bevorzugtesten im Bereich von pH 7 bis pH 10 und insbesondere im Bereich von pH 8 bis pH 9 auf. Der Begriff "pH-Optimum" ist dem Fachmann allgemein bekannt und betrifft jenen pH-Bereich, bei dem ein Enzym seine maximale enzymatische Wirksamkeit aufweist. In diesem Zusammenhang kann auf die einschlägige Literatur, wie beispielsweise Voet et al. "Biochemie", 1992, VCH-Verlag, Kapitel 13, Seite 331 verwiesen werden. Vorzugsweise ist im Sinne der Beschreibung unter dem pH-Optimum jener pH-Bereich zu verstehen, in dem die erfindungsgemäß verwendete Amidohydrolase wenigstens 80%, vorzugsweise wenigstens 90% der maximalen enzymatischen Wirksamkeit, bei ansonst gleichbleibenden Reaktionsbedingungen, aufweist.

Es wurde überraschend gefunden, dass Amidohydrolasen, d.h. Asparaginasen, bereitgestellt werden können, welche über einen sehr breiten pH-Bereich aktiv sind. Im Bereich von pH 5 - pH 10 weisen diese Amidohydrolasen, d.h. Asparaginasen, bevorzugt eine Aktivität von wenigstens 10% der maximalen Aktivität auf. Dadurch wird der Einsatz dieser Enzyme in verschiedenen Prozessen mit stark unterschiedlichen pH-Bereichen möglich. Auch ist ein Einsatz in Prozessen möglich, bei denen der pH-Wert im Prozess starken Schwankungen unterliegt. Auch sind insbesondere Prozesse möglich, in denen pH-Werte von 5 bis 10 auftreten. Bei der Behandlung von grünen Kaffeebohnen mit Leitungswasser beispielweise können sehr niedrige pH-Werte von - 5 auftreten.

In einer bevorzugten Ausführungsform weist die erfindungsgemäße Amidohydrolase, d.h. Asparaginase, über den gesamten pH-Bereich von 5-10 eine Aktivität von wenigstens 10%, bevorzugter wenigstens 15%, noch bevorzugter wenigstens 20%, am bevorzugtesten wenigstens 25% und insbesondere wenigstens 30% im Vergleich zu maximalen Aktivität auf, d.h. zur maximalen Aktivität bei optimalem pH-Wert unter ansonsten identischen Bedingungen, vorzugsweise bei optimaler Temperatur und Konzentration.

Die erfindungsgemäß verwendete Amidohydrolase, d.h. Asparaginase, ist vorzugsweise lagerstabil. In bevorzugten Ausführungsformen G₁-G₁₇ bis K₁-K₁₇ weist die Amidohydrolase unter den in der nachstehenden Tabelle angegebenen Bedingungen eine Restaktivität von wenigstens 80%, bevorzugter wenigstens 85%, noch bevorzugter wenigstens 90% und insbesondere wenigstens 95% auf:

| | | Temperaturbereich (°C) | | | | |
|---|---|---|---|---|---|---|
| | Lagerungsdauer | G | H | I | J | K |
| 1 | 5 Tage | 25 | 15 | 10 | 8 | 5 |
| 2 | 10 Tage | 25 | 15 | 10 | 8 | 5 |
| 3 | 15 Tage | 25 | 15 | 10 | 8 | 5 |
| 4 | 20 Tage | 25 | 15 | 10 | 8 | 5 |
| 5 | 25 Tage | 25 | 15 | 10 | 8 | 5 |
| 6 | 30 Tage | 25 | 15 | 10 | 8 | 5 |
| 7 | 60 Tage | 25 | 15 | 10 | 8 | 5 |
| 8 | 90 Tage | 25 | 15 | 10 | 8 | 5 |
| 9 | 120 Tage | 25 | 15 | 10 | 8 | 5 |
| 10 | 150 Tage | 25 | 15 | 10 | 8 | 5 |
| 11 | 180 Tage | 25 | 15 | 10 | 8 | 5 |
| 12 | 210 Tage | 25 | 15 | 10 | 8 | 5 |
| 13 | 240 Tage | 25 | 15 | 10 | 8 | 5 |
| 14 | 270 Tage | 25 | 15 | 10 | 8 | 5 |
| 15 | 300 Tage | 25 | 15 | 10 | 8 | 5 |
| 16 | 330 Tage | 25 | 15 | 10 | 8 | 5 |
| 17 | 360 Tage | 25 | 15 | 10 | 8 | 5 |

In vorstehender Tabelle bedeutet beispielsweise Ausführungsform I₅, dass die Amidohydrolase nach Lagerung für 25 Tage bei 10°C eine Restaktivität von wenigstens 80%, bevorzugter wenigstens 85%, noch bevorzugter wenigstens 90% und insbesondere wenigstens 95% aufweist. In einer besonders bevorzugten Ausführungsform der weist die erfindungsgemäß verwendete Amidohydrolase bei einer Lagerung bei 4°C über einen Zeitraum von 30 Tagen eine Restaktivität von wenigstens 80% auf.

Die Gruppe der Amidohydrolasen umfasst unter anderem die Enzymfamilie der Asparaginasen (EC 3.5.1.1), welche die Hydrolyse von Asparagin zu Aspartat und Ammoniak katalysieren. Bei der erfindungsgemäß verwendeten Amidohydrolase handelt es sich um eine Asparaginase.

Es ist allgemein bekannt, dass Asparaginasen sowohl Asparagin (L- und D-Form) als auch Glutamin (L- und D-Form) umsetzen können.

Angesichts dieser Substratpromiskuität von Asparaginasen hydrolysiert die erfindungsgemäß eingesetzte Asparaginase L-Asparagin vorzugsweise schneller als L-Glutamin und/oder ggf. schneller als D-Asparagin.

Vorzugsweise liegt das Verhältnis der K_{M}-Werte (jeweils in mM) von L-Asparagin : L-Glutamin im Bereich von 1:10 bis 1:400, bevorzugter im Bereich von 1:20 bis 1:200, noch bevorzugter im Bereich von 1:30 bis 1:100, am bevorzugtesten im Bereich von 1:40 bis 1:80.

In einer bevorzugten Ausführungsform präferiert die erfindungsgemäß eingesetzte Asparaginase L-Asparagin mehr als L-Glutamin und/oder mehr als D-Asparagin. Vorzugsweise liegt das Verhältnis der K_{M}-Werte (jeweils in mM) von L-Asparagin : L-Glutamin im Bereich von 1:1 bis 1:400, bevorzugter im Bereich von 1:5 bis 1:100, noch bevorzugter im Bereich von 1:10 bis 1:50.

Amidohydrolasen bzw. Asparaginasen können thermolabil oder thermostabil sein. Thermostabile Asparaginasen sind bereits aus dem Stand der Technik bekannt (vgl. z.B. Li et al., Anal. Chem. 2002, 74, p.3336-3341, US 5,719,056 oder Agathi et al., Mol. Cell. Biochem. 2001, 216, p.93-101). Hinweise auf deren Verwendung bei Prozessen der Zu- oder Aufbereitung von Nahrungsmitteln oder Genussmitteln können diesen Druckschriften jedoch nicht entnommen werden.

Unter den Begriffen "thermostabile Amidohydrolase" oder "thermostabile Asparaginase" im Sinne der Beschreibung ist vorzugsweise eine Amidohydrolase bzw. Asparaginase zu verstehen, welche nach einer Inkubationsdauer von 5 min bei 50°C eine Restaktivität von wenigstens 75% aufweist.

Vorzugsweise handelt es sich bei der Asparaginase um eine Asparaginase aus *Archaeoglobus* sp. *(z.B. Archaeoglobus fulgidus), Thermus sp. (z.B. Thermus thermophilus), Pyrococcus* sp. *(z.B. Pyrococcus abyssi), Thermococcus* sp. *(z. B. Thermococcus kodakarensis), Methanothermobacter* sp. *(z.B. Methanothermobacter thermautotrophicus)* oder um eine Asparaginase aus weiteren Euryarchaeota oder um die Asparaginase I von *Pyrococcus furiosus.*

In einer bevorzugten Ausführungsform ist die Asparaginase durch eine Nukleotidsequenz kodiert, welche vorzugsweise wenigstens 60%, bevorzugter wenigstens 80%, noch bevorzugter wenigstens 90%, noch bevorzugter wenigstens 95%, am bevorzugtesten wenigstens 99% und insbesondere wenigstens 99,9% homolog zur Nukleotidsequenz <SEQ ID NO: 1> ist. Die Homologie wird hierbei bevorzugt mit Hilfe des Algorithmus nach Smith & Waterman (J Mol Biol., 1981, 147(1), 195-7) ermittelt, unter Verwendung der BLOSUM62-Matrix und Werten von 11.0 für die Eröffnung einer Lücke, bzw. 1.0 für die Erweiterung einer Lücke.

Insbesondere ist es bevorzugt, dass die erfindungsgemäß verwendete Asparaginase durch die Nukleotidsequenz <SEQ ID NO: 1> kodiert ist.

Die Aminosäuresequenz der Asparaginase weist eine Homologie (Sequenzidentität) von wenigstens 50%, bevorzugter wenigstens 75%, noch bevorzugter wenigstens 80%, noch bevorzugter wenigstens 90%, noch bevorzugter wenigstens 95%, am bevorzugtesten wenigstens 99% und insbesondere wenigstens 99,9% zur Aminosäuresequenz <SEQ ID NO: 2> auf. Die Homologie wird hierbei bevorzugt mit Hilfe des Algorithmus nach Smith & Waterman (J Mol Biol., 1981, 147(1), 195-7) ermittelt, unter Verwendung der BLOSUM62-Matrix und Werten von 11.0 für die Eröffnung einer Lücke, bzw. 1.0 für die Erweiterung einer Lücke.

In einer anderen, besonders bevorzugten Ausführungsform umfasst die erfindungsgemäß verwendete Asparaginase die Aminosäuresequenz <SEQ ID NO: 2>.

Zu-/Aufbereitungsverfahren von vorzugsweise kohlenhydrathaltigen Nahrungs- oder Genussmitteln weisen, wie bereits vorstehend ausgeführt wurde, häufig Vorbehandlungsschritte zur Reduktion des Acrylamidgehalts in diesen Nahrungsmitteln oder Genussmitteln auf. Es ist daher bevorzugt, dass die erfindungsgemäße Verwendung der Amidohydrolase bei der Aufbereitung, insbesondere im Rahmen einer Vorbehandlung, zur Hydrolyse von Asparagin zu Asparaginsäure dient.

Unter dem Begriff "kohlenhydrathaltige Nahrungsmittel" sind im Sinne der Beschreibung vorzugsweise Nahrungsmittel zu verstehen, deren Gehalt an Kohlenhydraten vorzugsweise wenigstens 0,1 Gew.-%, bevorzugter wenigstens 1 Gew.-%, noch bevorzugter wenigstens 5 Gew.-%, noch bevorzugter wenigstens 10 Gew.-%, am bevorzugtesten wenigstens 20 Gew.-%, und insbesondere wenigstens 30 Gew.-%, bezogen auf das Gesamtgewicht des Nahrungsmittels beträgt.

Ferner ist es bevorzugt, dass die erfindungsgemäße Verwendung einer Amidohydrolase bei der Aufbereitung, insbesondere im Rahmen einer Vorbehandlung, der Verringerung des Gehalts an Asparagin und/oder Acrylamid in dem Nahrungsmittel oder dem Genussmittel dient.

Durch eine derartige, erfindungsgemäße Verwendung einer Amidohydrolase erfolgt vorzugsweise eine Verringerung des Gehalts an Asparagin, damit das Nahrungsmittel oder das Genussmittel bei einer thermischen Nachbehandlung einen verringerten Gehalt an Acrylamid aufweist.

Unter dem Begriff "thermische Nachbehandlung" sind im Sinne der Beschreibung bevorzugt jene Prozesse zu verstehen, die eine Erwärmung des Nahrungs- oder Genussmittels mit sich bringen. Übliche thermische Nachbehandlungen umfassen ein Erwärmen, Rösten, Grillen, Kochen, Garen, Backen, Dämpfen, Frittieren und dergleichen.

Besonders bevorzugt eignet sich die erfindungsgemäß eingesetzte Amidohydrolase bei Zu- oder Aufbereitungsprozessen von kohlenhydrathaltigen Nahrungsmitteln, wie beispielsweise Reis, Brot- und Backwaren, Snacks, Fertigmischungen, Trockenfrüchten, Tierfutter, etc. oder Genussmitteln, wie Kaffee oder Kakao. Derartige Nahrungs- und/oder Genussmittel sind vorzugsweise ausgewählt aus der Gruppe bestehend aus Knäckebrot, Zwieback, Keksen, Brezeln, Toastbrot, Waffeln, Muffins, Beagles, Croissants, Brownies, Frühstückscerealien, Biskuites, Kartoffelchips, Tortillachips, Maischips, Crackem, Nüssen, Pommes frites, Reiswaffeln, Polenta, Couscous, Pfannkuchen, Fertigmischungen, Kuchenmischungen, Biscuitmischungen, Brotmischungen, Croutons, Hundefutter, Katzenfutter, Kaffeebohnen, Kakaobohnen.

Besonders bevorzugt handelt es sich um Kaffebohnen. Bevorzugte Kaffeebohnenspezies sind *Coffea arabica, Coffea canephora, Coffea liberica und Coffea robusta.*

In einer besonders bevorzugten Ausführungsform umfasst die Aufbereitung eines Genussmittels eine Entkoffeinierung und/oder ein Waschen von Kaffeebohnen, bei der die erfindungsgemäße Amidohydrolase eingesetzt wird.

In einer ebenfalls besonders bevorzugten Ausführungsform umfasst die Aufbereitung eines Genussmittels eine Hydrolyse von Kaffeebohnen, die mit dem Einsatz der erfindungsgemäßen Amidohydrolase, vorzugsweise Asparaginase, kombiniert wird. Kaffeebohnen werden im Zuge der Vorbereitung einer Entkoffeinierung oder zur Geschmacks-Veredelung einer Wasserdampf-Behandlung unterzogen. Hierbei werden die Bohnen stark erhitzt und mit Wasser in Verbindung gebracht. Hier erweist sich der Einsatz einer thermoaktiven Amidohydrolase zur Reduktion derAsparagin-Konzentration in der grünen Kaffeebohne als besonders vorteilhaft. Der Enzym-Einsatz bei Temperaturen über 70°C ermöglicht eine vollständige Kompatibilität mit etablierten Verfahren und darüber hinaus sehr hohe Reaktionsgeschwindigkeiten, was die Prozesszeiten stark verkürzt.

Durch den erfindungsgemäßen Einsatz einer Amidohydrolase, d.h. einer Asparaginase, welche die vorstehend beschriebenen Eigenschaften aufweist, ergeben sich zahlreiche, überraschende Vorteile, die nachfolgend anhand von vier illustrativen Beispielen dargelegt werden. Ein Fachmann erkennt, dass derartige Beispiele in keiner Weise limitierend aufzufassen sind, da die erfindungsgemäße Amidohydrolase für einen Einsatz in einer Vielzahl von Aufbereitungsverfahren von Nahrungsmitteln oder Genussmitteln geeignet ist.

So werden üblicherweise bei der Aufbereitung von Kartoffelchips die Kartoffeln, bevor sie unter Dampf gekocht werden, mit Asparaginase vorbehandelt, d.h. die Kartoffeln werden in Scheiben geschnitten und die Asparaginase kann entweder aufgesprüht werden oder die Kartoffelscheiben werden in eine asparaginasehaltige Lösung getaucht. Die Dauer einer derartigen, herkömmlichen Asparaginasebehandlung kann dabei teilweise beträchtlich (bis zu mehrere Stunden) sein, da aufgrund des Temperatur-Optimums des Enzyms (üblicherweise 37°C, vgl. z.B. US 7,037,540, Beispiel 5) die Behandlungstemperatur maximal 37°C betragen darf und der Aufschluss von Asparagin entsprechend langsam verläuft.

Durch die thermostabilen Eigenschaften der erfindungsgemäß verwendeten Amidohydrolase können die Kartoffelscheiben bei höheren Temperaturen einer Asparaginasebehandlung unterzogen werden, d.h. die Asparaginreduktion erfolgt schneller, u.a. da die Löslichkeit des Asparagins bei höheren Temperaturen zunimmt. Teilweise kann die Asparaginasebehandlung, bedingt durch die thermostabilen Eigenschaften des Enzyms sogar gleichzeitig während des Kochens der Kartoffeln unter Dampf erfolgen. Die Asparaginase kann hierbei beispielsweise zuvor auf die Kartoffelscheiben gesprüht werden.

Eine Deaktivierung der Amidohydrolase erfolgt auf herkömmliche Weise durch eine thermische Nachbehandlung, d.h. durch Frittieren der Kartoffelscheiben. Gesundheitliche Bedenken hinsichtlich der Verwendung dieser thermostabilen Amidohydrolasen ergeben sich daher nicht.

Ein weiteres Beispiel betrifft die Entkoffeinierung von Kaffeebohnen. Bei diesen Aufbereitungsverfahren werden die ungerösteten, grünen Kaffeebohnen bedampft und/oder in z.T. heißem Wasser eingeweicht, um das Koffein aus den Bohnen zu extrahieren. Mit Hilfe der erfindungsgemäß eingesetzten Amidohydrolase wird es ermöglicht, derartige Entkoffeinierungsschritte, die üblicherweise bei Temperaturen über 37°C ablaufen, mit einer gleichzeitigen Asparaginasebehandlung zu kombinieren, d.h. der Aufbereitungsprozess von entkoffeiniertem Kaffee als solcher wird weniger zeitaufwändig und in der Folge kostengünstiger. Herkömmliche Verfahren zur Entkoffeinierung von Kaffeebohnen sind dem Fachmann bekannt und umfassen unter anderem das "Schweizer Wasserverfahren", die "direkte Methode", die "indirekte Methode" oder das "Triglyceridverfahren". In diesem Zusammenhang kann beispielsweise vollumfänglich verwiesen werden auf R. Heiss, Lebensmitteltechnologie: Biotechnologische, chemische, mechanische und thermische Verfahren der Lebensmittelverarbeitung, Springer; 6. Auflage, 2003.

Überraschenderweise kann die erfindungsgemäße Amidohydrolase auch besonders vorteilhaft bei einem Bedampfen/Befeuchten von Kaffeebohnen eingesetzt werden. Ein derartiges Bedampfen/Befeuchten dient, wie bereits vorstehend ausgeführt, zum Öffnen der Poren der Kaffeebohnen, um anschließend leichter das in den Bohnen vorhandene Asparagin zu inaktivieren/reduzieren. Ein Bedampfen/Befeuchten erfolgt üblicherweise bei erhöhten Temperaturen, d.h. bei Temperaturen bis vorzugsweise höchstens 100°C, da sowohl die Löslichkeit von Asparagin in warmen Lösungsmitteln erhöht ist, als sich auch die Poren der Kaffeebohnen bei warmen Temperaturen rascher öffnen. Mit Hilfe der erfindungsgemäßen, thermostabilen Amidohydrolase kann eine Reduktion von Asparagin einerseits gleichzeitig während des Bedampfens/Befeuchtens erfolgen, andererseits kann die Asparaginreduktion aufgrund der hohen Temperaturverträglichkeit des Enzyms schneller und effizienter ablaufen.

Die erfindungsgemäße Amidohydrolase kann auch bei der Herstellung von Frischbackwaren wie Brot, Brötchen oder dergleichen eingesetzt werden. Diese Frischbackwaren werden häufig mit Hilfe von Kochextrusions-Verfahren hergestellt, welche üblicherweise bei Temperaturen zwischen 95 und 105°C ablaufen. Durch die thermostabilen Eigenschaften der erfindungsgemäßen Amidohydrolase kann das Enzym während der Kochextrusion zugegeben werden und so eine Reduktion des Asparagingehalts bewirken. Für die Asparaginreduktion förderlich ist im Falle der Kochextrusion sowohl das Kneten des Teigs als auch die Entstehung von Gasblasen, welche durch das Entweichen von Kohlensäure aus dem erhitzten Wasser entstehen. Durch den anschließenden Back- bzw. Röstprozess, der üblicherweise in einem Temperaturbereich von 200 bis 600°C abläuft, wird die erfindungsgemäße Amidohydrolase inaktiviert bzw. denaturiert.

In vielen Behandlungsschritten von Nahrungs- und Genussmitteln spielen Inkubation in wässriger Umgebung bei hohen Temperaturen zwischen 70 und 110°C eine Rolle, die der thermischen Nachbehandlung, die zur Acrylamid-Bildung führt, vorgeschaltet sind. Hierzu zählen beispielweise Kochschritte. Wässrige Behandlungen in dem obigen Temperaturbereich werden zum Beispiel bei der Produktion von geformten Pommes frites oder von geformten Kartoffelchips eingesetzt. Der unerwartete Vorteil der erfindungsgemäßen Asparaginase liegt darin, dass das Enzym direkt in diesen Prozessen eingesetzt werden kann und bei den hohen Temperaturen sehr hohe Reaktionsgeschwindigkeiten erzielt, die mit sehr geringen Enzymmengen sehr hohe Wirksamkeiten ermöglichen.

Auch in Getreide-verarbeitenden Prozessen kann die erfindungsgemäße Amidohydrolase vorteilhaft eingesetzt werden. Bei der Herstellung von Corn Flakes wird Maisgrits im Gemisch mit Zucker, Salz und Malz gekocht und im Anschluss weiterverarbeitet und geröstet. Das Rösten verursacht die ungewollte Bildung des Acrylamids. Beim Kochen werden Temperaturen zwischen 70 und 100°C durchlaufen, die für einen Einsatz der erfindungsgemäßen Amidohydrolase sehr gut geeignet sind, so dass die Acrylamid-Bildung unterdrückt werden kann.

Bei Getreide-verarbeitenden Prozessen spielen Extrusions-Prozesse sehr häufig eine Rolle. Zum Beispiel werden bei der Produktion von Frühstücks-Cerealien Extrusions-Prozesse bei 80-100°C Endtemperatur durchgeführt. Auch sind Prozesse beschrieben, bei denen Halteprozesse bei hohen Temperaturen zwischen 70 und 100°C eine Rolle spielen.

Produkte, die bei hohen Temperaturen prozessiert werden und die Roggen enthalten, weisen in der Regel sehr hohe Acrylamid-Gehalte auf (zum Beispiel Knäckebrot). Bei dem Herstellen von Knäckebrot werden im Backprozess sehr schnell hohe Temperaturen erreicht. Das Knäckebrot kann vor dem Backprozess mit einer Lösung der erfindungsgemäßen Amidohydrolase behandelt werden, um die Acrylamid-Bildung beim Backprozess zu reduzieren.

Ein weiterer, unerwarteter Vorteil der erfindungsgemäß eingesetzten, thermostabilen Amidohydrolase ist die Möglichkeit einer Wiederverwendung (Rezyklieren) des Enzyms. So kann die Amidohydrolase, welche durch ihre thermostabilen Eigenschaften auch bei erhöhten Temperaturen, d.h. bis zu vorzugsweise 100°C nicht denaturiert, nach deren Einsatz extrahiert oder auf anderem Wege abgetrennt und damit zur erneuten Anwendung eingesetzt werden. Eine derart rezyklisierte Amidohydrolaselösung kann mehrere, d.h. vorzugsweise wenigstens 1, 2, 3, 4 oder 5 Zyklen zur Reduktion von Asparagin durchlaufen.

Die Anwendung der erfindungsgemäß eingesetzten Amidohydrolasen ist vorzugsweise gesundheitlich unbedenklich, da es sich bei diesen Amidohydrolasen um natürliche, nicht-toxische Substanzen handelt.

Ein weiterer Aspekt der vorliegenden Erfindung betrifft ein Verfahren zur Aufbereitung eines Nahrungsmittels oder eines Genussmittels umfassend die Schritte:
(i) Inkubieren des Nahrungsmittels oder des Genussmittels mit einer, wie vorstehend definierten Amidohydrolase bei einer Inkubationstemperatur von wenigstens 70°C, bevorzugter wenigstens 80°C, noch bevorzugter wenigstens 90°C und am bevorzugtesten wenigstens 99°C; und
(iii) ggf. Erwärmen des Nahrungsmittels oder des Genussmittels auf eine Temperatur, welche vorzugsweise wenigstens 10°C, bevorzugter wenigstens 15°C, noch bevorzugter wenigstens 20°C, am bevorzugtesten wenigstens 50°C und insbesondere wenigstens 60°C oberhalb der Inkubationstemperatur liegt.

In einer bevorzugten Ausführungsform umfasst das erfindungsgemäße Verfahren zur Aufbereitung eines Nahrungsmittels oder eines Genussmittels die Schritte:
(i) Inkubieren des Nahrungsmittels oder des Genussmittels mit einer, wie vorstehend definierten Amidohydrolase bei einer Inkubationstemperatur von we-nigstens 70°C, bevorzugter wenigstens 80°C, noch bevorzugter wenigstens 90°C und am bevorzugtesten wenigstens 99°C;
(ii) Abtrennen der Amidohydrolase von dem Nahrungsmittel oder dem Genussmittel oder Inaktivieren der Amidohydrolase;
(iii) ggf. Erwärmen des Nahrungsmittels oder des Genussmittels auf eine Temperatur, welche vorzugsweise wenigstens 10°C, bevorzugter wenigstens 15°C, noch bevorzugter wenigstens 20°C, am bevorzugtesten wenigstens 50°C und insbesondere wenigstens 60°C oberhalb der Inkubationstemperatur liegt; und
(iv) ggf. Wiedereinsetzen der ggf. in Schritt (ii) abgetrennten Amidohydrolase in Schritt (i).

Schritt (i) des erfindungsgemäßen Verfahrens wird vorzugsweise unter Bedingungen durchgeführt (Zeit, Temperatur, pH-Wert, Menge an Amidohydrolase, etc.), dass die ursprünglich in dem Nahrungs- oder Genussmittel enthaltene Menge an (freiem) Asparagin um wenigstens 50%, bevorzugter wenigstens 75%, noch bevorzugter wenigstens 80%, noch bevorzugter wenigstens 85%, am bevorzugtesten wenigstens 90% und insbesondere wenigstens 95% vermindert wird. Geeignete Bedingungen kann ein Fachmann durch übliche Routineversuche auffinden.

Schritt (i) des erfindungsgemäßen Verfahrens wird vorzugsweise unter Bedingungen durchgeführt (Zeit, Temperatur, pH-Wert, Menge an Amidohydrolase, etc.), dass die in der gegebenenfalls folgenden thermischen Nachbehandlung gebildete Acrylamid-Menge um wenigstens 20%, bevorzugt 30% noch bevorzugter um wenigstens 40% und am bevorzugtesten um wenigstens 50% reduziert wird.

In einer bevorzugten Ausführungsform wird Schritt (i) des erfindungsgemäßen Verfahrens vorzugsweise unter Bedingungen durchgeführt (Zeit, Temperatur, pH-Wert, Menge an Amidohydrolase, etc.), dass nach Durchführung von Schritt (iii) die in dem Nahrungs- oder Genussmittel enthaltene Menge an Acrylamid höchstens 200 ppm, bevorzugter höchstens 150 ppm, noch bevorzugter höchstens 135 ppm, am bevorzugtesten höchstens 100 ppm und insbesondere höchstens 50 ppm beträgt. Geeignete Bedingungen kann ein Fachmann durch übliche Routineversuche auffinden.

In einer ebenfalls bevorzugten Ausführungsform wird Schritt (i) des erfindungsgemäßen Verfahrens vorzugsweise unter Bedingungen durchgeführt (Zeit, Temperatur, pH-Wert, Menge an Amidohydrolase, etc.), dass nach Durchführung von Schritt (iii) die in dem Nahrungs- oder Genussmittel enthaltene Menge an Acrylamid höchstens 1.000 µg/kg, bevorzugter höchstens 500 µg/kg, noch bevorzugter höchstens 300 µg/kg, am bevorzugtesten höchstens 150 µg/kg und insbesondere höchstens 50 µg/kg beträgt. Geeignete Bedingungen kann ein Fachmann durch übliche Routineversuche auffinden.

In einer bevorzugten Ausführungsform erfolgt die Durchführung von Schritt (i) für wenigstens 240 min, bevorzugter wenigstens 120 min, noch bevorzugter wenigstens 60 min, am bevorzugtesten wenigstens 20 min und insbesondere wenigstens 5 min.

In einer bevorzugten Ausführungsform liegt das Gewichtsverhältnis von Nahrungs- oder Genussmittel zu Amidohydrolase im Bereich von 10² : 1 bis 10¹⁰ : 1, bevorzugter von 10² : 1 bis 10⁸ : 1, noch bevorzugter von 10⁴ : 1 bis 10⁸ : 1, am bevorzugtesten von 10⁴ : 1 bis 10⁷ : 1 und insbesondere von 10⁵ : 1 bis 10⁷ : 1.

In einer bevorzugten Ausführungsform wird die Amidohydrolase in einer wässrigen Lösung bereitgestellt und mit dem Nahrungs- oder Genussmittel zusammengebracht, beispielsweise durch Aufsprühen. Dabei beträgt die Konzentration der Amidohydrolase in der wässrigen Lösung bevorzugt 10⁻⁶ bis 100 g/l bevorzugter 10⁻⁵ bis 10 g/l, noch bevorzugter 10⁻⁴ bis 1 g/l, am bevorzugtesten 10⁻³ bis 10⁻¹ g/l und insbesondere 10⁻² bis 5 × 10⁻² g/l.

Ein weiterer Aspekt der Erfindung betrifft ein Nahrungsmittel oder Genussmittel, welches durch das vorstehend beschriebene Verfahren erhältlich ist. Das Nahrungsmittel oder Genussmittel weist vorzugsweise einen Restgehalt von höchstens 200 ppm, bevorzugter höchstens 150 ppm, noch bevorzugter höchstens 135 ppm, am bevorzugtesten höchstens 100 ppm und insbesondere höchstens 50 ppm an Asparagin und/oder Acrylamid auf.

Ein weiterer Aspekt der Erfindung betrifft ein Nahrungsmittel oder Genussmittel, welches durch das vorstehend beschriebene Verfahren erhältlich ist. Das Nahrungsmittel oder Genussmittel weist vorzugsweise einen Restgehalt von höchstens 400 µg/kg, bevorzugter höchstens 300 µg/kg, noch bevorzugter höchstens 200 µg/kg, am bevorzugtesten höchstens 100 µg/kg und insbesondere höchstens 50 µg/kg an Asparagin und/oder Acrylamid auf.

Ein weiterer Aspekt der Erfindung betrifft einen Vektor, welcher eine Nukleotidsequenz, wie vorstehend definiert, enthält. Vorzugsweise ist ein derartiger Vektor ausgewählt aus der Gruppe bestehend aus Plasmiden, Cosmiden, Phagemiden, Phagen-Vektoren, *Bacterial Artificial Chromosomes* und *Yeast Artificial Chromosomes.*

Ein weiterer Aspekt der Erfindung ist ein Verfahren zur Herstellung einer Amidohydrolase wie vorstehend definiert, das folgende Schritte **u**mfasst:
a) Einbringen eines Vektors, wie vorstehend definiert, in ein Expressionssystem;
b) ggf. Exprimieren der Amidohydrolase in dem Expressionssystem;
c) ggf. Aufschließen oder Lysieren oder Abtrennung des Expressionssystems;
d) ggf. Zugeben einer geeigneten, ggf. thermostabilen Nuclease zur Hydrolyse der Nucleinsäuren des Expressionssystems;
e) ggf. Denaturieren des Expressionssystems durch eine Inkubation bei vorzugsweise 60°C, bevorzugter 70°C, noch bevorzugter 80°C, am bevorzugtesten 90°C und insbesondere 100°C für die Dauer von vorzugsweise 1 min, bevorzugter 5 min, noch bevorzugter 10 min, am bevorzugtesten 20 min und insbesondere 60 min;
f) ggf. Abtrennen unerwünschter Bestandteile des Expressionssystems durch Zentrifugation, Filtration, Mikrofiltration oder Ultrafiltration;
g) ggf. Binden der Amidohydrolase an einen festen Träger, wobei die Bindung über ionische, hydrophobe oder Affinitäts-Tag vermittelte Wechselwirkungen erfolgt;
h) ggf. Waschen des Trägers zur Entfernung unerwünschter Bestandteile unter Bedingungen, bei denen die Amidohydrolase im wesentlichen am Träger gebunden bleibt;
i) ggf. Eluieren der Amidohydrolase vom Träger durch geeignete Pufferbedingungen;
k) ggf. Konzentrieren der Amidohydrolase-Lösung durch Fällung, Ultrafiltration, Gefriertrocknung oder Trocknung; und
l) ggf. Überführen der Amidohydrolase in einen geeigneten Lagerungspuffer.

Natürliche Asparaginasen können intrazellulär und extrazellulär lokalisiert werden. Will man Asparaginasen intrazellulär in großen Mengen überexprimieren, so ist man mit dem Problem konfrontiert, dass hohe Asparaginase-Konzentrationen in der Zelle einen toxischen Effekt ausüben, da sie die für die Zelle essentielle Aminosäure Asparagin hydrolysieren. Es wurde überraschend gefunden, dass die erfindungsgemäße Amidohydrolase in dem Temperaturbereich von 20-37°C, in dem üblicherweise die Fermentationsprozesse mesophiler Organismen wie zum Beispiel *Escherichia coli, Pseudomonas sp., Bacillus sp., Pichia pastoris, Saccharomyces cerevisiae* oder *Aspergillus sp.* durchgeführt werden, nur noch eine sehr geringe Rest-Aktivität aufweist. So hat das Enzym bei 25°C eine Restaktivität von < 2%. Dies ermöglicht die intrazelluläre Expression des erfindungsgemäßen Asparaginase in mesophilen Expressionswirten in großen Mengen.

Ein weiterer Aspekt der Erfindung umfasst ein Verfahren zur Herstellung einer Amidohydrolase wie vorstehend definiert, bei dem die Amidohydrolase intrazellulär in einem Mikroorganismus exprimiert wird, wobei die Restaktivität des Enzyms bei der Kultivierungs-Temperatur während der Expression der Amidohydrolase < 30% ist, bevorzugt < 15%, noch bevorzugter < 10%, noch bevorzugter < 5%, am bevorzugtesten < 2%.

Dem Fachmann zugängliche Fermentationsprotokolle für Mikroorganismen erlauben Ausbeuten an Biofeuchtmasse von bevorzugt >100 g/l, noch bevorzugter >125 g/l. noch bevorzugter >150 g/l , noch bevorzugter >175 g/l und am bevorzugtesten >200 g/l.

In einer bevorzugten Ausführungsform wird bei der intrazellulären Expression der Amidohydrolase in einem Mikroorganismus eine Aktivitätsausbeute von >10 kU / g Biofeuchtmasse, bevorzugter >20 kU / g Biofeuchtmasse, bevorzugter >40 kU / g Biofeuchtmasse, bevorzugter >60 kU / g Biofeuchtmasse, am bevorzugtesten >80 kU / g Biofeuchtmasse erreicht.

In einer bevorzugten Ausführungsform wird bei der intrazellulären Expression der Amidohydrolase in einem Mikroorganismus eine Aktivitätsausbeute von > 100.000 Units pro Liter Kulturvolumen, bevorzugt > 500.000 Units pro Liter, noch bevorzugter > 1 Mio. Units pro Liter, noch bevorzugter > 3 Mio. Units pro Liter, noch bevorzugter > 6 Mio. Units pro Liter und am bevorzugtesten > 10 Mio. Units pro Liter erreicht.

In einer bevorzugten Ausführungsform erfolgt die Expression der erfindungsgemäßen intrazellulär in einem mesophilen Expressionswirt wie zum Beispiel *Escherichia coli, Pseudomonas sp., Bacillus sp., Pichia pastoris, Saccharomyces cerevisiae* oder *Aspergillus sp.*

Die nachfolgenden Beispiele dienen zur näheren Erläuterung der Erfindung, sind jedoch nicht einschränkend auszulegen.

### Beispiele

### Beispiel 1 - Expression einer thermostabilen Asparaginase

Mit den beiden Primem PF_Asnl_S (5'- ACCTGCGGTCTCGCATGAAAATTCTTC-TAATTGGGATGGG-3'; <SEQ ID NO: 3>) und PF_Asnl_A (5'- GGATCCCTGCA-GTTAATCTCTAAGCTCTCCAACTAG-3'; <SEQ ID NO: 4>) (beide Thermo Electron, Ulm) wurde das für die Asparaginase I aus Pyrococcus furiosus kodierende Gen (<SEQ ID NO: 1>) durch eine PCR aus *Pyrococcus furiosus* DSM 3638-DNA unter den nachfolgenden Bedingungen amplifiziert.

### 1.1 PCR-Bedingungen:

### PCR-Ansatz:

| | |
|---|---|
| 10 µl | 10x VENT-Puffer (NEB, Beverly, USA) |
| 2 µl | dNTPs (je 10 mM) |
| 100 pmol | Primer PF_Asnl_S <SEQ ID NO: 3> |
| 100 pmol | Primer PF_Asnl _A <SEQ ID NO: 4> |
| 1 µl | DNA von *Pyrococcus furiosus* DSM 3638 |
| 2 U | VENT-Polymerase (NEB) |
| ad 100 µl | H₂O dest. |

Temperaturprofil der PCR:

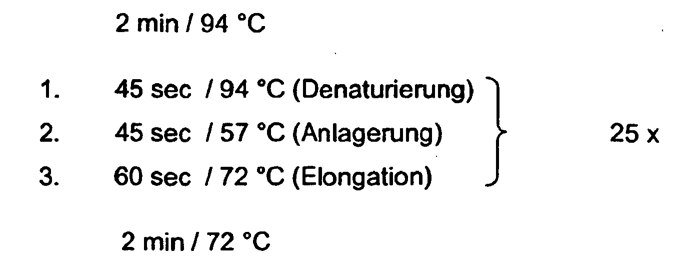

Das resultierende PCR-Produkt wurde über das QIAquick PCR-Reinigungs-Kit (Qiagen, Hilden) nach Herstellervorschrift aufgereinigt.

### 1.2 Restriktionsverdau:

Das unter Punkt 1.1 erhaltene Gen wurde in den Expressionsvektor pRSF-1b <SEQ ID NO: 5> kloniert (Vektor pRSF-1 b, Novagen-Merck-Biosciences, Bad Soden).

Dazu wurde das PCR-Produkt mittels der Restriktionsendonucleasen *Eco31I* und *PstI* und der Vektor pRSF-1 b <SEQ ID NO: 5> mittels der Restriktionsendonucleasen *NcoI* und *PstI* (alle Fermentas, Vilnius, Litauen) wie nachstehend aufgeführt verdaut:

### 1.3 Restriktionsverdau-Ansätze:

| PCR-Produkt | Vektor |
|---|---|
| 2 µg PCR-Produkt | 4 µg pRSF-1 b <SEQ ID NO: 5> |
| 3 µl 10x Puffer G⁺ (Fermentas) | 4 µl 10x Puffer Y⁺ (Fermentas) |
| 10 U *Eco31I* | 10 U *NcoI* |
| 20 U *PstI* | 20 U *PstI* |
| ad 30 µl H₂O dest. | ad 40 µl H₂O dest. |

Die Restriktionsverdau-Ansätze wurden 2 Stunden bei 37 °C inkubiert. Zu dem "Vektor-Ansatz" wurde anschließend zur Dephosphorylierung 1 U SAP (*Shrimp Alkaline Phosphatase,* Fermentas, Vilnius, Litauen) hinzu gegeben und für weitere 30 min bei 37 °C inkubiert. Anschließend wurden die Enzyme für 20 min bei 80 °C inaktiviert. Daraufhin wurden die Produkte mittels des QIAquick PCR-Reinigungs-Kit (Qiagen, Hilden) aufgereinigt.

### 1.4 Ligation, Transformation in E. coli und Plasmid-Reisolation

Die Vektor-DNA und das PCR-Produkt (vgl. Punkt 1.3) wurden durch die Inkubation mit T4-DNA-Ligase wie nachstehend ausgeführt miteinander verbunden:

### Ligase-Ansatz:

| | |
|---|---|
| 200 fmol | pRSF-1 b <SEQ ID NO: 5> |
| 600 fmol | PCR-Produkt |
| 3 µl | 10x Ligase-Puffer (Fermentas) |
| 1 µl | T4-DNA-Ligase |
| ad 30 µl | H₂O dest. |

Die Ansätze wurden 8 h bei 16 °C inkubiert und anschließend wurde das Enzym durch 10-minütige Inkubation bei 65 °C inaktiviert. 1 µl dieses Ansatzes wurde direkt zur Transformation kommerziell erhältlicher kompetenter XL1 Blue-Zellen (Stratagene, La Jolla, USA) mittels Elektroporation eingesetzt. Die elektroporierten Zellen wurden auf Festagar-Platten mit Kanamycin ausplattiert und über Nacht bei 37°C kultiviert. Ausgehend von einer resultierenden Einzelkolonie wurde das fertige Plasmid mittels des Plasmid-Reinigungskits QIAprep Minipräparations-Kit (Qiagen, Hilden) nach Herstellervorschrift reisoliert und das Expressions-Plasmid pRSF_Pf-Asnl <SEQ ID NO: 6> erhalten.

Das Expressions-Plasmid pRSF_Pf-Asnl <SEQ ID NO: 6> wurde mittels Elektroporation in Rosetta 2 (DE3) (Novagen-Merck-Biosciences, Bad Soden) Zellen eingebracht und die Zellen auf LB-Agar-Platten (10 g Trypton, 5 g Hefeextrakt, 10 g NaCl, ad 1 l Aqua dest.) mit Kanamycin (Kan) und Chloramphenicol (Cam) ausplattiert.

Von diesen Platten wurden Einzelklone gepickt und zunächst Vorkulturen für die Expression hergestellt. Dazu wurden 100 ml LB (Kan, Cam)-Medium mit 1% (w/v) Glucose mit 1 ml einer 5 ml Vorkultur angeimpft und bis zum Erreichen einer OD₆₀₀ von 0,6 bei 37 °C und 200 Upm (Umdrehungen pro Minute) geschüttelt. Die Zellen wurden abzentrifugiert (4 °C, 15 min, 3200xg) und der Überstand verworfen. Das Pellet wurde in 2 ml 10% (v/v) Glycerol resuspendiert. Die Suspension wurde zu je 200 µl aliquotiert, in flüssigem Stickstoff eingefroren und bei -80 °C gelagert.

Die Hauptkultur für die Expression bestand aus 500 ml LB (Kan, Cam)-Medium mit 1% (w/v) Glucose. Sie wurde mit einem Aliquot der Vorkultur angeimpft. Die Hauptkultur wurde bei 37°C und 200 Upm inkubiert. Nach Erreichen einer OD₆₀₀ von 0,9 wurde mit 1 mM IPTG (Isopropyl-β-D-thiogalactopyranosid) induziert und über Nacht bei 30 °C und 200 Upm geschüttelt. Am nächsten Tag wurden die Zellen durch Zentrifugation (4 °C, 15 min, 3200×g) sedimentiert und nach Entfernung des Mediums wurde das Pellet gewogen und mit 20 ml Lysepuffer (10 mM Tris/HCl pH 8,0, 0,5 mg/ml Lysozym) resuspendiert und mit Ultraschall aufgeschlossen (5×30 s, 80% Leistung). Die Suspension der aufgeschlossenen Zellen wurde zentrifugiert (4 °C, 30 min, 14000×g). Der Überstand wurde anschließend einer Inkubation für 30 min bei 80°C unterzogen und einer erneuten Zentrifugation unterworfen (4 °C, 30 min, 14000×g). Das Pellet wurde verworfen und der so gewonnene Rohextrakt wurde abgenommen und für weitere Untersuchungen bei 4 °C gelagert. Von dem Überstand wurde die Aktivitätsausbeute mit 160 kU bestimmt (vgl. Beispiel 2). Aus dem Gewicht des Zellpellets von 4 g ergabt sich eine Ausbeute von 40 kU pro g Biofeuchtmasse.

### Beispiel 2 - Bestimmung des Temperaturprofiles

### Assay Prinzip

Asparaginasen katalysieren die Umwandlung von Asparagin in Aspartat unter der Freisetzung von Ammonium-lonen. Diese können über Farbreagenzien, wie beispielsweise das Nessler-Reagens nachgewiesen. Alternativ können Ammonium-Ionen auch über die Berthelot-Reaktion (DIN 38 406 E5) nachgewiesen werden. Der Assay mit Nessler-Reagens beruht auf einer Endpunkt-Bestimmung. Die Reaktion wird dabei über einen Zeitraum von 30 min bei der entsprechenden Temperatur inkubiert, abgestoppt und die entstandenen Ammonium-lonen detektiert.

### Reagenzien und Lösungen

### Reagenzien:

L-Asparagin Monohydrat: Applichem A3669, MW 150,14 g/mol
Ammoniumsulfat: Merck, 1.101211, MW 132,14 g/mol
Nessler-Reagens: Fluka, 72190
Tris, TCA

### Stammlösungen und Puffer:

50 mM Tris/HCl pH 8,6
172 mM L-Asparagin-Lösung
1,5 M TCA (Trichloressigsäure)

### Eichlösungen:

5 mM (NH₄)₂SO₄-Lösung

### 2.1 Erstellung einer Eichkurve

Hierzu wurden folgende Ansätze gemacht:

| jeweils 50 µl | | | | | | 50 mM Tris/HCl pH 8,6 |
|---|---|---|---|---|---|---|
| 0 µl | 5 µl | 10 µl | 20 µl | 30 µl | 40 µl | 5 mM (NH₄)₂SO₄ |
| 45 µl | 40 µl | 35 µl | 25 µl | 15 µl | 5 µl | dest. H₂O |

Die Ansätze wurden gemischt und jeweils 5 µl 1,5 M TCA zugegeben und erneut gemischt.

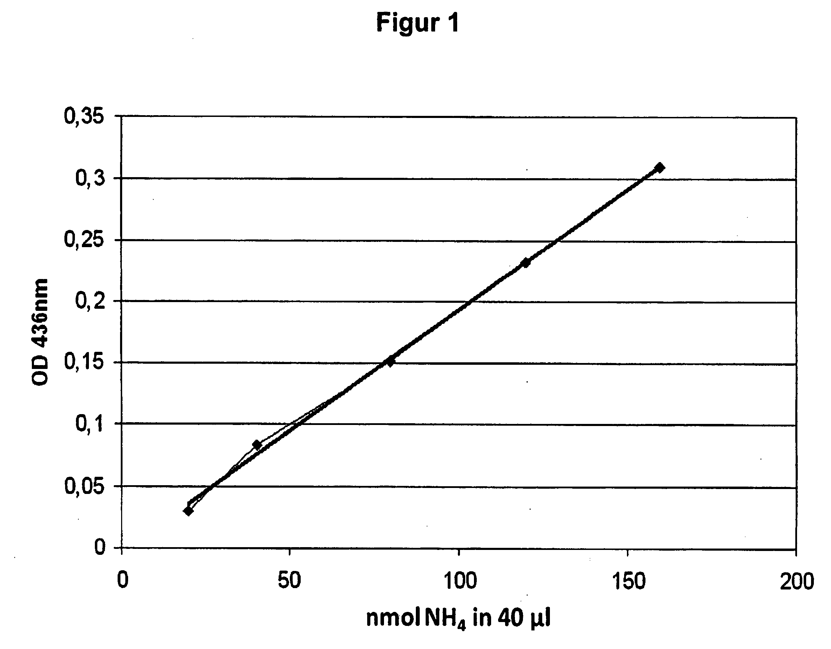

Es wurde für jeden Wert 860 µl dest. H₂O in einem 1,5 ml Reaktionsgefäß vorgelegt und 40 µl des jeweiligen Ansatzes zugegeben und gemischt. Zu jedem Ansatz wurden zügig 100 µl Nessler-Reagens gegeben und die Proben kurz gemischt. Nach 5 min wurde das Photometer bei 436 nm gegen Wasser abgeglichen und die Proben zügig vermessen. Die Werte wurden in eine Eichgerade eingetragen (vgl. Figur 1).

### 2.2 Probenmessung

Mittels eines kommerziellen Protein-Bestimmungstest (Bradfort, Bio-Rad Laboratories GmbH, München) wurde die Protein-Konzentration des Rohextraktes aus Ausführungsbeispiel 1 mit ca. 4 mg/ml bestimmt. Die Asparaginase-Lösung wurde 1:2000 in 50 mM Tris/HCl pH 8,6 verdünnt. Für jede zu messende Probe wurde folgender Ansatz vorgelegt:

| | |
|---|---|
| 50 µl | 50 mM Tris/HCl pH 8,6 |
| 35 µl | dest. H₂O |
| 5 µl | 172 mM L-Asn-Lösung |

Die Ansätze wurden gemischt und ein Thermocycler auf die gewünschte Temperatur (37, 70, 80, 90 und 99 °C) vorgeheizt.

Zu den Ansätzen wurden 5 µl der verdünnten Enzymprobe und zum Leerwert 5 µl Puffer gegeben, die Ansätze kurz gemischt und dann für 30 min bei der jeweiligen Reaktionstemperatur inkubiert. Anschließend wurden die Reaktionen auf Eis abgekühlt und durch Zugabe von 5 µl 1,5 M TCA-Lösung gestoppt.

Die Bestimmung der freigesetzten Ammonium-lonen erfolgte sofort im Anschluss. Es wurde für jeden Wert 860 µl dest. H₂O in einem 1,5 ml Reaktionsgefäß vorgelegt und 40 µl des jeweiligen Ansatzes zugegeben und gemischt. Zu jedem Ansatz wurden zügig 100 µl Nessler-Reagens gegeben und die Proben kurz gemischt. Nach 5 min wurde das Photometer bei 436 nm gegen Wasser abgeglichen und die Proben zügig vermessen.

Zuerst wurde der ermittelte Absorptionswert in freigesetzte Ammonium-lonen anhand der Eichkurve (vgl. Figur 1) umgerechnet. Anschließend wurde über die Definition der Unit (eine Unit Asparaginase setzt 1 µmol NH₄ pro min unter Assay-Bedingungen frei) die volumetrische Aktivität [U/ml] des Enzyms berechnet. Derartige Berechnungen sind dem Fachmann allgemein bekannt.

### 2.3 Bestimmung des Temperatur-Optimums

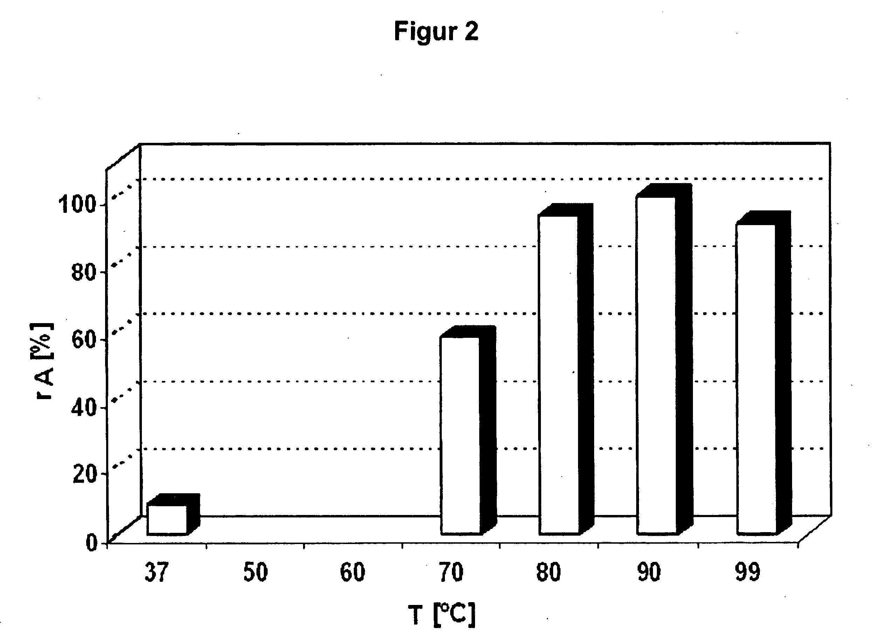

Für die verschiedenen Temperaturen wurde der Gehalt an NH₄ bei einer Wellenlänge von 436 nm gemessen und die daraus resultierenden volumetrischen (Enzym-) Aktivitäten ermittelt. Die volumetrische Aktivität bei 90 °C wurde 100% gesetzt (=Referenzwert) und die jeweiligen volumetrischen Aktivitäten bei den anderen Temperaturen entsprechend auf diesen Referenzwert bezogen (= relative Aktivität). Die entsprechenden Werte sind in der nachfolgenden Tabelle, sowie grafisch in Figur 2 zusammengefasst:

| Temp. | Messwert 436 nm | Units pro ml | Relative Aktivität |
|---|---|---|---|
| 37°C | 0,018 | 320 | 9% |
| 70°C | 0,126 | 2140 | 58% |
| 80°C | 0,205 | 3460 | 94% |
| 90°C | 0,218 | 3670 | 100% |
| 99°C | 0,2 | 3370 | 92% |

### Beispiel 3 - Bestimmung des Temperaturstabilität

Reagenzien und Lösungen, die Bestimmung der Eichkurve sowie die Durchführung der Aktivitätstests erfolgten wie vorstehend in Ausführungsbeispiel 2 beschrieben.

### 3.1 Temperaturstabilität bei 95°C

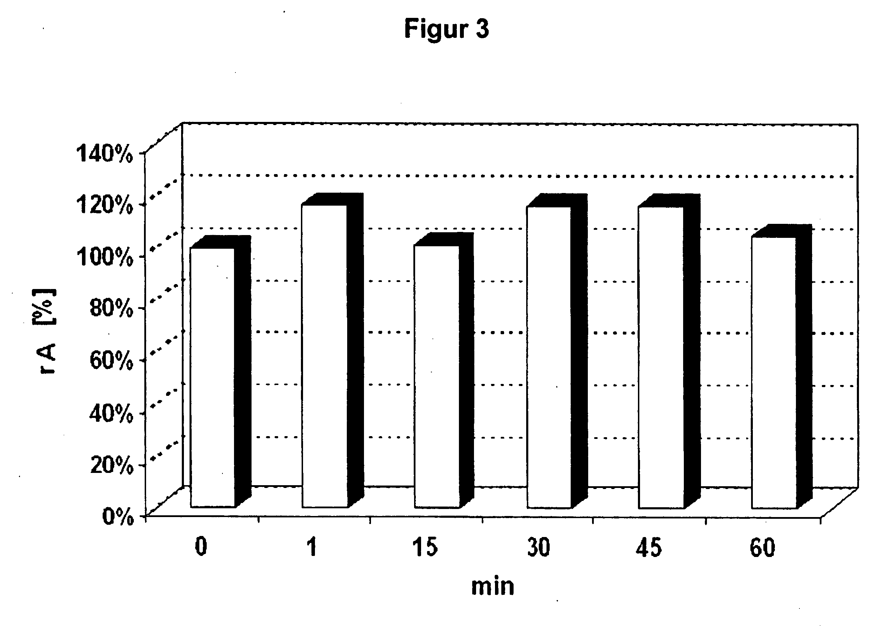

Die Bestimmung der volumetrischen (Enzym-)Aktivität erfolgte hierbei immer bei einer Inkubationstemperatur von 90°C. Für die Bestimmung der Temperaturstabilität wurde die Asparaginase-Lösung aus Ausführungsbeispiel 1 1:10 in 50 mM Tris/HCl pH 8,6 verdünnt und anschließend für verschiedene Zeitintervalle bei 95°C (0, 1, 15, 30, 45 und 60 min) vorinkubiert. Anschließend erfolgte eine weitere Verdünnung von 1:100 in 50 mM Tris/HCl pH 8,6 und die Bestimmung der verbliebenen Restaktivität. Die volumetrische enzymatische Aktivität ohne Vorinkubation bei 95°C wurde 100% gesetzt (=Referenzwert) und alle anderen Werte auf diesen Referenzwert bezogen (=relative Aktivität). Die dabei erhaltenen/errechneten Werte sind in der nachfolgenden Tabelle (vgl. auch Figur 3) zusammengefasst:

| Zeit in min | Messwert bei 436 nm | Units pro ml | Relative Aktivität |
|---|---|---|---|
| 0 | 0,398 | 3340 | 100% |
| 1 | 0,466 | 3900 | 117%* |
| 15 | 0,403 | 3380 | 101%* |
| 30 | 0,464 | 3890 | 116%* |
| 45 | 0,464 | 3890 | 116%* |
| 60 | 0,418 | 3500 | 105%* |

| | | | |
|---|---|---|---|
| * relative Aktivität = Restaktivität | | | |

### 3.2 Temperaturstabilität bei 99°C

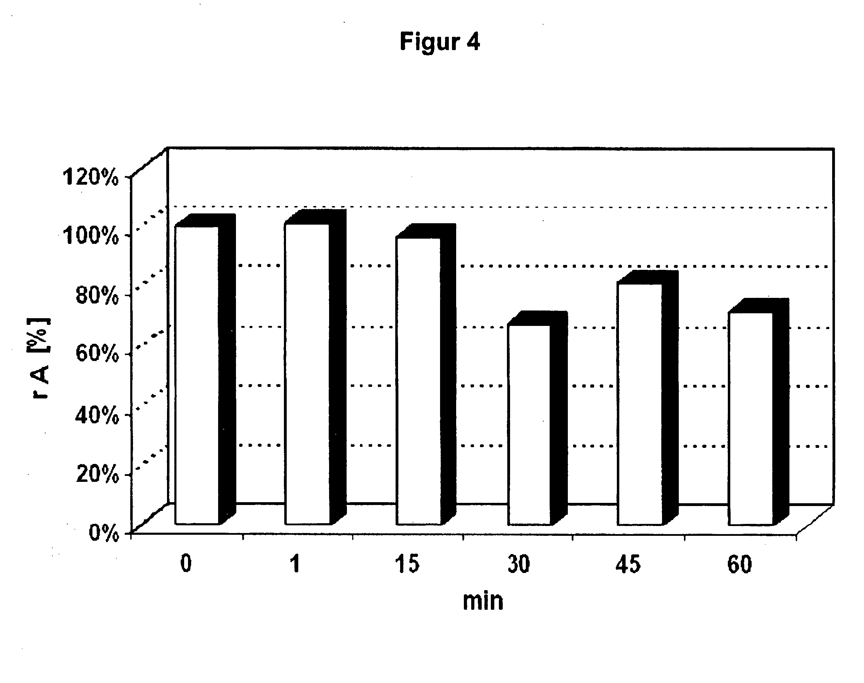

Der Versuch zur Bestimmung der Temperaturstabilität bei 99°C wurde analog zu dem vorstehend in Punkt 3.1 beschriebenen Versuch durchgeführt. Die hierbei erhaltenen/errechneten Werte sind in der nachstehenden Tabelle (vgl. auch Figur 4) zusammengefasst:

| Zeit in min | Messwert bei 436 nm | Units pro ml | Relative Aktivität |
|---|---|---|---|
| 0 | 0,411 | 3440 | 100% |
| 1 | 0,416 | 3490 | 101%* |
| 15 | 0,396 | 3320 | 97%* |
| 30 | 0,276 | 2320 | 67%* |
| 45 | 0,329 | 2790 | 81%* |
| 60 | 0,294 | 2470 | 72%* |

| | | | |
|---|---|---|---|
| * relative Aktivität = Restaktivität | | | |

### Beispiel 4 - Reduktion des Acrylamidgehaltes in Kaffeebohnen

Zum Nachweis der Wirksamkeit der Behandlung von Lebensmitteln mit der erfindungsgemäßen Asparaginase wurden Rohkaffeebohnen vor der Röstung einem Behandlungsschritt mit dem Enzym bei 80 °C unterzogen und die Reduktion des Acrylamidgehaltes nach der Röstung bestimmt.

Hierzu wurden folgende Versuchsansätze erstellt:

| | |
|---|---|
| Blank 1: | 500 g Rohkaffeebohnen Sorte Arabica-Mischung + |
| | 267 g 100 mM TRIS/HCl pH 8,6 (25 °C) |
| | |
| Blank 2: | 500 g Rohkaffeebohnen Sorte Brazil Arabica + |
| | 267 g 100 mM TRIS/HCl pH 8,6 (25 °C) |
| | |
| Probe 1: | 500 g Rohkaffeebohnen Sorte Arabica-Mischung + |
| | 267 g 100 mM TRIS/HCl pH 8,6 (25 °C) + |
| | 1400 Units Asparaginase aus Ausführungsbeispiel 1 |
| | |
| Probe 2: | 500 g Rohkaffeebohnen Sorte Brazil Arabica + |
| | 267 g 100 mM TRIS/HCl pH 8,6 (25 °C) + |
| | 1400 Units Asparaginase aus Ausführungsbeispiel 1 |

Die Blanks und Proben wurden 60 min bei 80 °C unter Rotation inkubiert. Anschließend wurde die Flüssigkeit von den Kaffeebohnen abfiltriert, die Bohnen getrocknet und geröstet.

Die Ermittlung des Acrylamidgehaltes erfolgte durch ein unabhängiges akkreditiertes Prüflabor. Die Ergebnisse der Untersuchungen zeigt nachfolgende Tabelle (vgl. auch Figur 4):

| | Acrylamidgehalt in µg/kg | Mittelabweichung in µg/kg | Restgehalt |
|---|---|---|---|
| Blank 1 | 480 | 20 | 100% |
| Probe 1 | 222 | 19 | 46% |
| | | | |
| Blank 2 | 585 | 35 | 100% |
| Probe 2 | 273 | 18 | 47% |

## Patentansprüche

1. Verwendung einer Amidohydrolase, welche nach einer Inkubationsdauer von 20 min bei 70°C eine Restaktivität von wenigstens 75% und ein Temperatur-Optimum im Bereich von 70 bis 120°C aufweist, zur Aufbereitung eines Nahrungsmittels oder eines Genussmittels durch Inkubieren des Nahrungsmittels oder Genussmittels mit der Amidohydrolase bei einer Inkubationstemperatur von wenigstens 70°C,
wobei die Amidohydrolase eine Asparaginase ist, deren Aminosäuresequenz eine Homologie von wenigstens 50% zur Aminosäuresequenz <SEQ ID NO: 2> aufweist.

2. Verwendung nach Anspruch 1, **dadurch gekennzeichnet, dass** die Amidohydrolase
- bei einer Temperatur im Bereich von 60 bis 120°C eine spezifische Aktivität von wenigstens 200 Units/mg aufweist; und/oder
- ein pH-Optimum im Bereich von pH 1 bis pH 14 aufweist; und/oder
- nach Lagerung bei 4°C über einen Zeitraum von 1 Monat eine Restaktivität von wenigstens 80% aufweist; und/oder
- über den pH-Bereich von pH 5 - pH 10 eine Aktivität von mindestens 10% im Vergleich zu maximalen Aktivität aufweist.

3. Verwendung nach einem der vorstehenden Ansprüche, **dadurch gekennzeichnet, dass** die Asparaginase "Asparaginase I" aus *Pyrococcus furiosus* ist.

4. Verwendung nach einem der vorstehenden Ansprüche, **dadurch gekennzeichnet, dass** die Asparaginase durch eine Nukleotidsequenz kodiert ist, welche wenigstens 60% homolog zur Nukleotidsequenz <SEQ ID NO: 1> ist.

5. Verwendung nach einem der vorstehenden Ansprüche, **dadurch gekennzeichnet, dass** die Aminosäuresequenz der Asparaginase eine Homologie von wenigstens 75% zur Aminosäuresequenz <SEQ ID NO: 2> aufweist.

6. Verwendung nach einem der vorstehenden Ansprüche, **dadurch gekennzeichnet, dass** die Aufbereitung des Nahrungsmittels oder des Genussmittels
- zur Hydrolyse von Asparagin zu Asparaginsäure dient; und/oder
- der Verringerung des Gehalts an Asparagin und/oder Acrylamid in dem Nahrungsmittel oder dem Genussmittel dient.

7. Verwendung nach Anspruch 6, **dadurch gekennzeichnet, dass** die Verringerung des Gehalts an Asparagin erfolgt, damit das Nahrungsmittel oder das Genussmittel bei einer thermischen Nachbehandlung einen verringerten Gehalt an Acrylamid aufweist.

8. Verwendung nach einem der vorstehenden Ansprüche, **dadurch gekennzeichnet, dass** das Nahrungsmittel und/oder Genussmittel ausgewählt ist aus der Gruppe bestehend aus Knäckebrot, Zwieback, Keksen, Brezeln, Toastbrot, Waffeln, Muffins, Beagles, Croissants, Brownies, Frühstückscerealien, Biskuites, Kartoffelchips, Tortillachips, Maischips, Crackem, Pommes frites, Reiswaffeln, Polenta, Couscous, Pfannkuchen, Nüssen, Fertigmischungen Kuchenmischungen, Biscuitmischungen, Brotmischungen, Croutons, Hundefutter, Katzenfutter, Kaffeebohnen und Kakaobohnen.

9. Verwendung nach Anspruch 8, **dadurch gekennzeichnet, dass** die Aufbereitung eine Entkoffeinierung und/oder ein Waschen von Kaffeebohnen umfasst.

10. Verfahren zur Aufbereitung eines Nahrungsmittels oder eines Genussmittels umfassend den Schritt:
(i) Inkubieren des Nahrungsmittels oder des Genussmittels mit einer Amidohydrolase definiert wie in einem der Ansprüche 1 bis 5 bei einer Inkubationstemperatur von wenigstens 70°C.

11. Verfahren nach Anspruch 10, **dadurch gekennzeichnet, dass** es den Schritt umfasst:
(ii) Abtrennen der Amidohydrolase von dem Nahrungsmittel oder dem Genussmittel oder Inaktivieren der Amidohydrolase.

## Claims

1. Use of an amidohydrolase, which after an incubation duration of 20 min at 70°C has a residual activity of at least 75% and a temperature optimum in the range of 70 to 120°C for preparing a foodstuff or a stimulant by incubating the foodstuff or the stimulant with the amidohydrolase at an incubation temperature of at least 70°C,
whereby the amidohydrolase is an asparaginase, the amino acid sequence of which has at least 50% homology with the amino acid sequence <SEQ ID NO: 2>.

2. Use according to claim 1, **characterized in that** the amidohydrolase
- has a specific activity of at least 200 units/mg at a temperature in the range of 60° to 120° C; and/or
- has a pH optimum in the range of pH 1 to pH 14; and/or
- after storage at 4°C over a period of 1 month has a residual activity of at least 80%; and/or
- has an activity of at least 10 % compared to its maximum activity over the pH range of pH 5 to pH 10.

3. Use according to any one of the preceding claims, **characterized in that**, the asparaginase is "asparaginase I" from *Pyrococcus furiosus.*

4. Use according to any one of the preceding claims, **characterized in that**, the asparaginase is coded by a nucleotide sequence, which has at least 60% homology with the nucleotide sequence <SEQ ID NO: 1>.

5. Use according to any one of the preceding claims, **characterized in that** the amino acid sequence of the asparaginase has a homology of at least 75% homology with the amino acid sequence <SEQ ID NO: 2>.

6. Use according to any one of the preceding claims, **characterized in that** preparing the foodstuff or the stimulant
- serves to hydrolyze asparagine to asparaginic acid; and/or
- leads to a reduction of the content of asparagine and/or acrylamide in the foodstuff or the stimulant.

7. Use according to claim 6 **characterized in that** the reduction in the content of asparagine occurs, so that the foodstuff or the stimulant has a reduced content of acrylamide during a thermal aftertreatment.

8. Use according to any one of the preceding claims, **characterized in that** the foodstuff and/or the stimulant is selected from the group consisting of crispbread, rusks, biscuits, pretzels, white toasting bread, waffles, muffins, bagels, croissants, brownies, breakfast cereals, biscotti, potato crisps, tortilla chips, corn chips, crackers, chips, rice cakes, polenta, couscous, pancakes, nuts, ready-mixed cake mixes, biscuit mixes, bread mixes, croutons, dog food, cat food, coffee beans and cocoa beans.

9. Use according to claim 8, **characterized in that** the preparation comprises decaffeinating and/or washing of coffee beans.

10. A process of preparing a foodstuff or a stimulant comprising the step:
(i) Incubating the foodstuff or the stimulant with an amidohydrolase as defined in any one of claims 1 to 5 at an incubation temperature of at least 70°C.

11. Process according to claim 10, **characterized in that**, the process comprises the step:
(ii) Separating the amidohydrolase from the foodstuff or the stimulant or inactivating the amidohydrolase.

## Revendications

1. Utilisation d'une amidohydrolase, qui, après une durée d'incubation de 20 min à 70 °C, présente une activité résiduelle d'au moins 75 % et un optimum de température dans la plage de 70 à 120 °C, pour le traitement d'un produit alimentaire ou d'un autre produit de consommation par incubation du produit alimentaire ou de l'autre produit de consommation avec l'amidohydrolase à une température d'incubation d'au moins 70 °C,
l'amidohydrolase étant une asparaginase, dont la séquence d'acides aminés présente une homologie d'au moins 50 % avec la séquence d'acides aminés <SEQ ID N° : 2>.

2. Utilisation selon la revendication 1, **caractérisée en ce que** l'amidohydrolase
- présente à une température dans la plage de 60 à 120 °C une activité spécifique d'au moins 200 unités/mg ; et/ou
- présente un optimum de pH dans l'intervalle de pH 1 à pH 14 ; et/ou
- présente après stockage à 4 °C pendant une durée de 1 mois une activité résiduelle d'au moins 80 % ; et/ou
- présente dans l'intervalle de pH de pH 5 - 10 une activité d'au moins 10 % par comparaison avec l'activité maximale.

3. Utilisation selon l'une quelconque des revendications précédentes, **caractérisée en ce que** l'asparaginase est l'« asparaginase I » de *Pyrococcus furiosus.*

4. Utilisation selon l'une quelconque des revendications précédentes, **caractérisée en ce que** l'asparaginase est codée par une séquence de nucléotides qui présente une homologie d'au moins 60 % avec la séquence de nucléotides <SEQ ID N° : 1>.

5. Utilisation selon l'une quelconque des revendications précédentes, **caractérisée en ce que** la séquence d'acides aminés de l'asparaginase présente une homologie d'au moins 75 % avec la séquence d'acides aminés <SEQ ID N° : 2>.

6. Utilisation selon l'une quelconque des revendications précédentes, **caractérisée en ce que** le traitement du produit alimentaire ou de l'autre produit de consommation
- sert à l'hydrolyse de l'asparagine en acide aspartique ; et/ou
- sert à diminuer la teneur en asparagine et/ou acrylamide du produit alimentaire ou de l'autre produit de consommation.

7. Utilisation selon la revendication 6, **caractérisée en ce que** la diminution de la teneur en asparagine s'effectue afin que le produit alimentaire ou l'autre produit de consommation présente dans le cas d'un post-traitement thermique une teneur réduite en acrylamide.

8. Utilisation selon l'une quelconque des revendications précédentes, **caractérisée en ce que** le produit alimentaire et/ou l'autre produit de consommation est/sont choisi(s) dans le groupe constitué par le pain croquant suédois, la biscotte, les gâteaux secs, les bretzels, le pain de mie, les gaufres, les muffins, les bagels, les croissants, les brownies, les céréales pour petit déjeuner, les biscuits, les chips de pomme de terre, les chips tortilla, les chips de maïs, les crackers, les pommes de terre frites, les galettes de riz, la polenta, le couscous, les crêpes, les noix, les préparations prêtes à l'emploi, les mélanges prêts à l'emploi pour gâteaux, les préparations prêtes à l'emploi pour biscuits, les mélanges de panification, les croûtons, les aliments pour chiens, les aliments pour chats, les grains de café et les fèves de cacao.

9. Utilisation selon la revendication 8, **caractérisée en ce que** le traitement comprend une décaféinisation et/ou un lavage de grains de café.

10. Procédé pour le traitement d'un produit alimentaire ou d'un autre produit de consommation, comprenant l'étape :
(i) incubation du produit alimentaire ou de l'autre produit de consommation avec une amidohydrolase telle que définie dans l'une quelconque des revendications 1 à 5, à une température d'incubation d'au moins 70 °C.

11. Procédé selon la revendication 10, **caractérisé en ce qu'**il comprend l'étape :
(ii) séparation de l'amidohydrolase d'avec le produit alimentaire ou l'autre produit de consommation ou inactivation de l'amidohydrolase.
